(19) Europäisches Patentamt
European Patent Office
Office européen des brevets

(11) EP 2 855 360 B1

(12) # EUROPEAN PATENT SPECIFICATION

(45) Date of publication and mention of the grant of the patent:
**28.02.2018 Bulletin 2018/09**

(21) Application number: **13797062.0**

(22) Date of filing: **28.05.2013**

(51) Int Cl.:
*C01G 55/00* (2006.01) *C07F 17/00* (2006.01)
*G01N 21/65* (2006.01) *B82Y 15/00* (2011.01)
*G01N 33/532* (2006.01) *G01N 33/543* (2006.01)

(86) International application number:
**PCT/SG2013/000215**

(87) International publication number:
**WO 2013/180652 (05.12.2013 Gazette 2013/49)**

(54) **SURFACE ENHANCED RAMAN SPECTROSCOPY (SERS) MARKER CONJUGATES AND METHODS OF THEIR PREPARATION**

MARKERKONJUGATE FÜR OBERFLÄCHENVERSTÄRKTE RAMAN-SPEKTROSKOPIE UND VERFAHREN ZU DEREN HERSTELLUNG

CONJUGUÉS DE MARQUEUR DE SPECTROSCOPIE RAMAN EXALTÉE DE SURFACE (SERS) ET LEURS PROCÉDÉS DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2012 SG 201204008**

(43) Date of publication of application:
**08.04.2015 Bulletin 2015/15**

(73) Proprietors:
- **Agency for Science, Technology and Research Singapore 138632 (SG)**
- **Nanyang Technological University Singapore 639798 (SG)**

(72) Inventors:
- **KONG, Kien Voon Singapore 138667 (SG)**
- **OLIVO, Malini Singapore 138667 (SG)**
- **LEONG, Weng Kee Singapore 637371 (SG)**
- **LAM, Zhiyong Singapore 637371 (SG)**

(74) Representative: **Viering, Jentschura & Partner mbB
Patent- und Rechtsanwälte
Kennedydamm 55 / Roßstrasse
40476 Düsseldorf (DE)**

(56) References cited:
**US-A1- 2012 128 592**

- **LI, C. ET AL.: 'Osmium Carbonyl Clusters on Gold and Silver Nanoparticles as Models for Studying the Interaction with the Metallic Surface' J. PHYS. CHEM. C vol. 113, 2009, pages 18562 - 18569, XP055179547**
- **MONTGOMERY, H. J. ET AL.: 'Examination of the Silver Colloid Binding Behavior of Disulfide-Tethered Bipyridine Ligands and Their fac-Tricarbonylrhenium(I) Complexes' INORG. CHEM. vol. 50, 2011, pages 2738 - 2747, XP055179548**
- **WANG, S. ET AL.: 'Au Nanoparticles Encapsulated in Ru Carbonyl Carboxylate Shells' LANGMUIR vol. 22, 2006, pages 7861 - 7866, XP055179549**
- **TAN, H. ET AL.: ''Preparation and Characterization of Cr(CO)4dpp (Chromium Tetracarbonyl 2,3-Bis(2'-pyridyl)pyrazine) Adsorbed on Silver Nanoparticles'' J. PHYS. CHEM. B vol. 109, 2005, pages 19657 - 19663, XP055179550**
- **KONG, K. V. ET AL.: 'Metal Carbonyl-Gold Nanoparticle Conjugates for Live-Cell SERS Imaging' ANGEW. CHEM. INT. ED. vol. 51, 2012, pages 9796 - 9799, XP055183614**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 855 360 B1

## Description

### TECHNICAL FIELD

The invention lies in the field of spectroscopy and molecular diagnostics. In particular, the disclosure refers to surface enhanced Raman spectroscopy (SERS) marker conjugates and methods for producing the SERS marker conjugates.

### BACKGROUND

Vibrational spectroscopic techniques, such as infra-red (IR), normal Raman spectroscopy and surface enhanced Raman spectroscopy (SERS), have been developed for analyte detection.

Even though these spectroscopic techniques may be considered well-established with readily available instrumentation, there remain unresolved issues regarding use of these techniques in biomedical research and applications. For example, a major hindrance to IR detection for live cells imaging relates to interference of the generated spectra from a strong absorption peak of water at about 1,600 $cm^{-1}$. Comparatively, Raman spectroscopy is able to provide better spatial resolution with minimal interference from water. However, it suffers from a low scattering cross section of only about $10^{-31}$ to $10^{-26}$ $cm^2$ per molecule. This lower scattering cross section necessitates use of a higher concentration of biotags for analyte detection, which results in cytotoxicity hence cell death, thereby limiting use of Raman spectroscopy in clinical applications.

SERS has evolved as one of the most sensitive techniques for analyte detection due to enhancement of the Raman spectral intensity by interaction of the adsorbed SERS active analyte molecules with surface of a metal substrate. In SERS, Raman signals of adsorbed molecules on colloidal gold or silver nanoparticles may be enhanced by several orders of magnitude, typically in the $10^6$ to $10^{14}$ range, due to strong surface plasmon resonance of the nanostructured surface. This has been successfully adapted for chemical sensing applications, at lower concentrations but with better detection limits, and is exemplified by its use in DNA detection, cancer diagnosis, and cellular molecules detection.

Current state of the art methods to form SERS nanotags include immobilizing a Raman active dye (Raman reporter) on a metal colloidal particle. The SERS nanotags formed are bioconjugated to specific locations on a target analyte. Such a nanoparticle-Raman reporter assembly may also be termed a Raman tag, and may provide a platform for multiplexing, targeting and tracking in bioimaging and sensing applications. The types of reporter molecules and metal nanoparticles are major determinants of the sensitivity of a Raman tag. Examples of reporter molecules include triphenylmethine (TM) compounds, such as malachite green isothiocyanate (MGITC) and crystal violet (CV).

Li, C. et al ("Osmium Carbonyl Clusters on Gold and Silver Nanoparticles as Models for Studying the Interaction with the Metallic Surface", J. Phys. Chem. C 2009, 113, 18562-18569) discloses the production of osmium carbonyl clusters that interact with silver and gold nanoparticles either directly by the cluster core, or by a spacer carrying a free thiol, alcohol or carboxylic acid functional group (title and abstract). The mode of interaction between the cluster and the surface depends on the nature of the functional group used to bind to the surface and the identity and nature (nanoparticles or substrate) of the surface. In most cases, the clusters tend to interact with the metal surface via the functional group, viz., -SH, - OH, or -COOH.

Montgomery, H. J. et al ("Examination of the Silver Colloid Binding Behavior of Disulfide-Tethered Bipyridine Ligands and Their fac-Tricarbonylrhenium(I) Complexes") discloses silver colloid bound *fac*-tricarbonylrhenium(I) complexes used in Raman experiments (abstract). The spectra are dominated by the characteristic peaks of a metalated 2,20-bipyridyl group, arising from the silver colloid/ion complexation, and the rhenium center. The rhenium complexes show weak SERS bands related to the CO stretches and a broad band at 510 $cm^{-1}$ assigned to Re-CO stretching. Herein, it is envisaged that the excitation wavelength can be used to address specifically the alkyl chain, the bipyridine, or even the rhenium-centered metal-to-ligand charge-transfer (MLCT) part of the molecule because this should provide a direct route to switching output signals from the various parts of the molecule, allowing interrogation of both the chromophore and other appended components.

Wang, S. et al ("Au Nanoparticles Encapsulated in Ru Carbonyl Carboxylate Shells", Langmuir 2006, 22, 7861-7866) discloses a two-step surface functionalization approach to encase gold nanoparticles with ruthenium dodecacarbonyl clusters via a mercaptopropionic acid linker (abstract). The ruthenium dodecacarbonyl clusters bound to the gold nanoparticle surface exhibit a Raman signal in the region of 1900-2100 $cm^{-1}$ (Figure 2A).

Tan, H. et al ("Preparation and Characterization of Cr(CO)4dpp (Chromium Tetracarbonyl 2,3-Bis(2'-pyridyl)pyrazine) Adsorbed on Silver Nanoparticles", J. Phys. Chem. B 2005, 109, 19657-19663) discloses the preparation and characterization of chromium tetracarbonyl 2,3-bis(2'-pyridyl)pyrazine adsorbed on silver particles (abstract). The conjugate exhibits a SERS signal in the region of 1008-2004 $cm^{-1}$ (Table 1).

US 2012/0128592 A1 discloses the use of organic-based Raman reporter dyes (paragraphs [0008] and [0009]). These Raman-active marker compounds may be attached to the surface of a nanoparticle to form the SERS marker

conjugate (paragraph [0093]). The conjugate may be used as a biosensor for the detection of an analyte using SERS spectroscopy (paragraph [0098]).

The SERS nanotags are themselves capable of giving off signals under SERS and have signals in the 800 $cm^{-1}$ to 1800 $cm^{-1}$ region. This translates into peak overlapping between peaks generated from SERS reporters and analytes, as most signals of biomolecules are present in the same region. As a result, identification of the biomolecules using SERS is hindered.

In view of the above, there is a need for an improved compound that may be used for detecting an analyte using Surface Enhanced Raman Spectroscopy (SERS) that addresses at least one of the above-mentioned problems, as well as methods of forming the compound.

## SUMMARY

In a first aspect, there is provided a method for detecting one or more analytes by surface enhanced Raman spectroscopy (SERS) using a SERS marker conjugate comprising a metallic nanoparticle and an organometallic material comprising or consisting essentially of a metal carbonyl compound, the organometallic material being attached to a surface of the metallic nanoparticle by metal bonding between the metallic nanoparticle and metal atom comprised in the organometallic material, the method comprising a) contacting the one or more analytes with at least one analyte binding molecule attached to the SERS marker conjugate; and b) detecting a surface enhanced Raman signal from the SERS marker conjugate.

In a second aspect, there is provided a biosensor for the detection of an analyte, the biosensor comprising a plurality of SERS marker conjugates, each SERS marker conjugate comprising a metallic nanoparticle and an organometallic material comprising or consisting essentially of a metal carbonyl compound, wherein the organometallic material is attached to a surface of the metallic nanoparticle by metal bonding between the metallic nanoparticle and metal atom comprised in the organometallic material, wherein the biosensor further comprises a substrate with the nanoparticle being attached to or adherent to the substrate.

In one example, the disclosure refers to a method of forming a surface enhanced Raman spectroscopy (SERS) marker conjugate according to the method of the first aspect.

## BRIEF DESCRIPTION OF THE DRAWINGS

The disclosure will be better understood with reference to the detailed description when considered in conjunction with the non-limiting examples and the accompanying drawings, in which:

**FIG. 1** shows a schematic diagram depicting an approach to form a surface enhanced Raman spectroscopy (SERS) marker conjugate according to various embodiments. In the embodiment shown, a SERS marker conjugate of osmium carbonyl clusters coated gold nanoparticle (termed herein as an Os-Au nanoparticle or an OM-NP construct) is formed. $Os_3(CO)_{10}(\mu\text{-}H)_2$, which is an example of an organometallic material, or more specifically, a metal carbonyl cluster, is attached on a surface of a gold nanoparticle, which is an example of a metallic nanoparticle. Attachment of the organometallic material to the metallic nanoparticle may take place by metal-metal interaction between the metal atom of the organometallic material and the metallic nanoparticle, or by organic ligand-metal interaction between the organic ligand of the organometallic material and the metallic nanoparticle.

**FIG. 2(A) to (D)** shows a schematic diagram for preparing an organometallic material-metallic nanoparticle (OM-NP construct) according to an embodiment. In the embodiment shown in **FIG. 2(A),** an $Os_3(CO)_{10}(\mu\text{-}H)_2$ metal carbonyl cluster is shown. In **FIG. 2(B),** a plurality of the $Os_3(CO)_{10}(\mu\text{-}H)_2$ metal carbonyl clusters are attached on gold nanoparticles to form OM-NP constructs, using strong interactions between the $Os_3(CO)_{10}(\mu\text{-}H)_2$ clusters and gold nanoparticles. The OM-NP constructs formed are functionalized with binding ligands of EGFR antibodies (denoted as L in the figure) and polyethylene glycol (PEG) to form OM-NP(PEG)-L constructs. As can be seen from **FIG. 2(C),** the OM-NP(PEG)-L constructs show detectable CO signal (peak at about 2000 $cm^{-1}$) in aqueous solution. **FIG. 2(D)** shows addition of the OM-NP(PEG)-L constructs to a live cell for subsequent SERS analysis. For comparison purposes, **FIG. 2(E) to (F)** show a schematic diagram in which $Os_3(CO)_{10}(\mu\text{-}H)_2$ metal carbonyl clusters are dispersed in an aqueous solution. The aqueous solution comprising the $Os_3(CO)_{10}(\mu\text{-}H)_2$ metal carbonyl clusters does not have a detectable CO signal, as evidenced by absence of a peak at about 2000 $cm^{-1}$ in **FIG. 2(F).** The $Os_3(CO)_{10}(\mu\text{-}H)_2$ metal carbonyl clusters in **FIG. 2(E)** are not suitable to be added to a live cell for subsequent SERS analysis.

**FIG. 3(A)** shows SERS spectra of Os-Au nanoparticles (OM-NP constructs) and bioconjugated EGFR-PEG-Os-Au nanoparticles (OM-NP(PEG)-L constructs) in aqueous solution. Y-axis: counts; x-axis: Raman shift in $cm^{-1}$. As can be seen from the figure, there is detectable Raman scattering signal from Os-Au nanoparticles in aqueous solution. Peaks within the shaded region of about 1960 $cm^{-1}$ to 2120 $cm^{-1}$ are those of the reporter molecules, which do not

overlap with the live cell signals. **FIG. 3(B)** shows SERS spectra of (from top to bottom) 10 μM and 50 mM $Os_3(CO)_{10}(\mu-H)_2$ in ethanol:water (1:4, v/v) solution; OM-NP constructs; and OM-NP(PEG)-L constructs in aqueous solutions, respectively. Y-axis: counts, scale bar: 1000 counts; x-axis: Raman shift in $cm^{-1}$. Shaded region denotes signals from the reporter molecules which do not overlap with the analyte signals.

**FIG. 4(A) and (B)** show (A) graph of Raman intensity against time (day) for time course study of OM-NP constructs at 0, 7, 14, 21, and 28 days, and (B) graph of Raman intensity against time (day) for time course study of OM-NP(PEG)-L constructs at 0, 7, 14, 21, and 28 days in water. **FIG. 4(C) and (D)** show transmission electron microscopy (TEM) images of the OM-NP constructs at (C) Day 1 and (D) Day 30. Scale bar in (C) and (D) denotes a length of 50 nm.

**FIG. 5(A) and (B)** show UV spectra of (A) OM-NP; and (B) OM-NP(PEG)-L constructs. Successful conjugation of EGFR antibody to the OM-NP constructs is indicated by the ultraviolet absorbance at wavelength of 280 nm in (B) (shaded region).

**FIG. 6** shows graphs of time course study and spectra of (A) OM-NP and (B) OM-NP(PEG)-L constructs collected over 28 days in water.

**FIG. 7** shows a graph comparing between cell viability of OSCC cells after 24 hours incubation with $Os_3(CO)_{10}(\mu-H)_2$, gold nanoparticles (as control), OM-NP constructs, and OM-NP(PEG)-L constructs. The figure shows that, while the cluster $Os_3(CO)_{10}(\mu-H)_2$ is clearly cytotoxic, the constructs are not as the cells remained about 100 % viable with respect to the control.

**FIG. 8(A) to (H)** are bright field and SERS mapping images of OSCC (A to D) and SKOV cells (E to H) treated with OM-NP(PEG)-L constructs. All mapping images (2030 $cm^{-1}$) were scanned at an interval of 1 μm (633 nm excitation). Scale bar in the figures denote a length of 5 μm.

**FIG. 9(A) to (J)** are SERS spectra and images of OSCC cells (EGFR-positive) upon incubation with OM-NP(PEG)-L constructs: (A, B, F and G) bright-field images; (C and H) SERS mapping images; (D and I) dark-field images; SERS spectra on (E) Day 1 and (J) Day 3. All measurements were performed with excitation at 633 nm and a laser power of 6 mW. Scale bar in (A), (B), (F) and (G) denotes a length of 20 μm. Scale bar in (C) denotes a length of 7.5 μm. Scale bar in (H) denotes a length of 5 μm. Scale bar in (D) and (I) denotes a length of 10 μm.

**FIG. 10(A)** shows dark-field images of OSCC cells after incubation with OM-NP(PEG)-L constructs, and **(B)** SERS spectra of OSCC cells at the four different locations indicated in (A). Y-axis: counts, and x-axis: Raman shift in $cm^{-1}$. Scale bar in (A) denotes a length of 10 μm.

**FIG. 11** is a graph showing IR spectrum (absorbance mode) of $Os_3(CO)_{10}(\mu-H)_2$ in ethanol. Y-axis: absorbance; x-axis: wavenumber in $cm^{-1}$.

**FIG. 12** is a graph showing Beer's law plot for $Os_3(CO)_{10}(\mu-H)_2$ in ethanol; cell path length = 0.1 mm. Y-axis: Abs; x-axis: concentration in mM.

**FIG. 13** is a graph showing Raman spectrum of 50 mM solution of $Os_3(CO)_{10}(\mu-H)_2$ in ethanol:water (1:4, v/v). Y-axis: counts; x-axis: Raman shift in $cm^{-1}$.

**FIG. 14** is a graph showing SERS spectrum of OM-NP constructs suspended in water ($4.3 \times 10^{-5}$ μM). Y-axis: counts; x-axis: Raman shift in $cm^{-1}$.

**FIG. 15** shows molecular structures of Compounds 1a, 1b, 2a, 2b, 3 and 4.

**FIG. 16** shows SERS spectra of OM-NP conjugates of Compounds 1a, 1b, 2a, 2b, 3 and 4. Y-axis: counts; x-axis: Raman shift in $cm^{-1}$.

## DETAILED DESCRIPTION

Surface enhanced Raman spectroscopy (SERS) marker conjugates to be used in a method according to embodiments as disclosed herein are able to provide a unique SERS signal at a region of 1800 $cm^{-1}$ to 2200 $cm^{-1}$, thereby avoiding interference with signals emitted by biomolecules which are in the 800 $cm^{-1}$ to 1800 $cm^{-1}$ region. This allows identification of biomolecules without the need to decouple signals emitted from the SERS marker conjugates. Advantageously, a combination of the marker conjugates may be used to provide a more complex spectrum for multiplex detection. Furthermore, the SERS marker conjugates exhibit improved toxicological behavior as compared to state of the art compounds used in SERS analysis, and have excellent storage stability.

Accordingly, in a first aspect, the present disclosure refers to a method for detecting analytes by surface enhanced Raman spectroscopy (SERS) using a SERS marker conjugate. The SERS marker conjugate is also referred to herein as a nanotag or a SERS nanotag. The term "conjugate" as used herein refers to two or more molecules which have been linked together. The linkage to each other may be covalent or non-covalent.

The SERS marker conjugate according to the first aspect comprises a metallic nanoparticle and an organometallic material attached to a surface of the metallic nanoparticle. The term "organometallic material" as used herein refers to a compound that contains at least one bond between a metal atom and a carbon atom in an organic molecule, ion, or radical. The metal atom in the organometallic material is not limited to bonding with a carbon atom, and may, additionally or alternatively, bond to other atoms or another metal atom comprised in the organometallic material.

The organometallic material contains both a metal and an organic ligand. In various embodiments, the organometallic material includes a metal with metal-carbon single bonds or metal-carbon multiple bonds, as well as a metal complex with unsaturated molecules, such as metal-$\pi$-complexes; or compounds such as sandwich compounds including full sandwiches, half sandwiches, multidecker sandwiches such as triple decker sandwiches, and inverse sandwiches.

Generally, any organometallic material that is able to be attached to and/or interact with metallic nanoparticles may be used. Depending on the organometallic material used, differing SERS signals within the 1800 $cm^{-1}$ to 2200 $cm^{-1}$ region may be obtained.

The organometallic material may include more than one metal atom, such as 2, 3, 4 or 5 metal atoms. Each metal atom may be formed from the same metal element or different. The metal of the organometallic material may be an alkali metal, an alkaline earth metal, an inner transition metal (a lanthanide or actinide), a transition metal, or a post-transition metal. In various embodiments, the metal of the organometallic material comprises a metal selected from the group consisting of magnesium, aluminum, titanium, vanadium, chromium, manganese, iron, cobalt, nickel, zirconium, ruthenium, hafnium, tantalum, tungsten, rhenium, osmium, or a combination comprising at least one of the metals listed herein.

In various embodiments, the metal in the organometallic material is a transition metal. Examples of transition metal include metals in Group 3 to 12 of the Periodic Table of Elements, such as titanium (Ti), vanadium (V), niobium (Nb), tantalum (Ta), chromium (Cr), molybdenum (Mo), tungsten (W), manganese (Mn), iron (Fe), ruthenium (Ru), osmium (Os), iridium (Ir), nickel (Ni), and copper (Cu).

In various embodiments, the organometallic material comprises a metal carbonyl compound. The organometallic material may consist essentially or consists of a metal carbonyl compound. The term "metal carbonyl compound" as used herein refers to coordination complexes of transition metals with carbon monoxide. The metal carbonyl compounds may have general formula $M_x(CO)_y$, where M denotes a metal, CO denotes a carbonyl ligand, and x and y are integers. x may have a value in the range from about 1 to 10, such as 1, 2, 3, 4, 5, 6, 7, 8, 9 or 10. In some embodiments, x is in the range from 1 to 4, such as 1, 2, 3 or 4. Value of y is determined by the total valence of the metal atom and may have a value in the range from 2 to 40, such as from 2 to 30, 2 to 24, 2 to 12, or 4 to 10. Generally, y has a value greater than x. Examples of metal carbonyls include, but are not limited to carbonyls formed from osmium, molybdenum, tungsten, or ruthenium. Specific examples of metal carbonyls may be $Mo(CO)_6$, $W(CO)_6$, $Mn_2(CO)_9$, $Cr(CO)_6$, $Co(CO)_8$, or derivatives thereof.

The metal carbonyl may comprise more than one metal. For example, M in the general formula $M_x(CO)_y$ mentioned above may be represented by $M_1M_2$, where $M_1$ and $M_2$ denote different metals. In such embodiments, the metal carbonyl compound may have general formula $(M_1M_2)_x(CO)_y$, wherein $M_1$ and $M_2$ denote different metals, and CO, x and y having the same definitions as that mentioned above.

The metal carbonyl compound may be homoleptic, i.e. contain only CO ligands, but may also contain a mix of different ligands besides carbonyl ligands. In such embodiments, the metal carbonyl compound may have general formula $L_mM(CO)_n$, wherein L denotes a ligand; m and n are integers; and M and CO having the same definitions as that mentioned above. m may have a value in the range from about 1 to 4, such as 1, 2, 3 or 4. Value of n is determined by the total valence of the metal atom and may have a value in the range from 1 to 5, such as 1, 2, 3, 4, or 5.

Examples of ligands include, but are not limited to, cyclopentadienyl (Cp), cyclobutadiene, cyclooctadiene, cyclooctatetraene, phosphate ligands, ethylene, halides such as chloride and iodide, phosphines, phosphites, amines, arsines, stibenes, ethers, sulfides, alkylidenes, nitrites, isonitriles, thiocarbonyls, linear, branched, or cyclic monoalkenes, linear, branched, or cyclic dienes, linear, branched, or cyclic trienes, bicyclic alkenes, bicyclic dienes, bicyclic trienes, tricyclic alkenes, tricyclic dienes, tricyclic trienes, alkynes, and the like.

The ligands of the organometallic material may be unsubstituted or substituted. As used herein "substituted" refers to a compound or radical substituted with at least one (e.g., 1, 2, 3, 4, 5, 6 or more) substituents independently selected from a halide (e.g., $F^-$, $Cl^-$, $Br^-$, $I^-$), a hydroxyl, an alkoxy, a nitro, a cyano, an amino, an azido, an amidino, a hydrazino, a hydrazono, a carbonyl, a carbamyl, a thiol, a $C_1$ to $C_6$ alkoxycarbonyl, an ester, a carboxyl, or a salt thereof, sulfonic acid or a salt thereof, phosphoric acid or a salt thereof, a $C_1$ to $C_{20}$ alkyl, a $C_2$ to $C_{16}$ alkynyl, a $C_6$ to $C_{20}$ aryl, a $C_7$ to $C_{13}$ arylalkyl, a $C_1$ to $C_4$ oxyalkyl, a $C_1$ to $C_{20}$ heteroalkyl, a $C_3$ to $C_{20}$ heteroaryl (i.e., a group that comprises at least one aromatic ring, wherein at least one ring member is other than carbon), a $C_3$ to $C_{20}$ heteroarylalkyl, a $C_3$ to $C_{20}$ cycloalkyl, a $C_3$ to $C_{15}$ cycloalkenyl, a $C_6$ to $C_{15}$ cycloalkynyl, a $C_5$ to $C_{15}$ heterocycloalkyl, or a combination including at least one of the moieties listed herein, instead of hydrogen, provided that the substituted atom's normal valence is not exceeded.

In alternate embodiments, the ligands may be inorganic, for example, $CO_2$, and CN, in their neutral or ionic forms.

In specific embodiments, the ligand comprises a substituted or unsubstituted cyclopentadienyl ligand, for example $C_5H_5$ or $C_5Me_5$, wherein Me denotes a methyl group. However, any further Cp ligands with one or more different ligands may also be used. For example, one or more of the hydrogen atoms of $C_5H_5$ may be substituted with ethyl, Cp or phenyl. In some embodiments, a mixture of ligands is used. For example, in addition to a cyclopentadienyl ligand, the metal carbonyl compound may further comprise a halide such as chloride or iodide, and tricyclohexyl phosphine.

The metal carbonyl compounds may be prepared by a variety of methods. For example, metals such as nickel, iron, cobalt, molybdenum and tungsten may react with carbon monoxide to form the respective metal carbonyls. Other methods for forming metal carbonyls include synthesis of carbonyls from salts and oxides in the presence of a suitable reducing agent such as copper, aluminium, hydrogen, lithium aluminum hydride and carbon monoxide. As an example, chromium hexacarbonyl ($Cr(CO)_6$) may be prepared from anhydrous chromium(III) chloride ($CrCl_3$) in benzene using aluminum chloride as catalyst, and aluminum as reducing agent.

For the preparation of higher molecular weight species such as polynuclear and heteronuclear metal carbonyls, condensation of lower molecular weight metal carbonyls may be used. For example, polynuclear and heteronuclear metal carbonyls may be synthesized using a condensation process involving either a reaction induced by coordinatively unsaturated species, or a reaction between coordinatively unsaturated species in different oxidation states.

The organometallic material may have two, three or more metal atoms, and may assume the form of an organometallic cluster or metal carbonyl cluster. Accordingly, the metal carbonyl compound that is used to form the SERS marker conjugate may comprise or consist essentially of metal carbonyl clusters. As used herein, the term "metal carbonyl cluster" refers to metal cluster compounds comprising carbon monoxide in complex combination with metal atoms, wherein the metal atoms in the metal carbonyl cluster are held together entirely or at least substantially by bonds between metal atoms.

Carbonyl ligands and/or other ligands such as those mentioned above (for example, cyclopentadienyl) may be bonded to some or all of the metal atoms to form a complex. In some embodiments, a carbonyl ligand is bonded to two metal atoms to form a bridge between the two metal atoms. Other suitable bridging groups may include, for example, phosphine, arsine and mercapto groups. Examples of metal carbonyl clusters include, but are not limited to, iron nonacarbonyl ($Fe_2(CO)_9$), cyclopentadienyliron dicarbonyl dimer $[Cp_2Fe(CO)_2]_2$, tetracobalt dodecacarbonyl ($Co_4(CO)_{12}$), ruthenium carbonyl ($Ru_3(CO)_{12}$), hexarhodium hexadecacarbonyl ($Rh_6(CO)_{16}$), osmium carbonyl ($Os_3(CO)_{12}$), iridium carbonyl ($Ir_4(CO)_{12}$), and rhenium carbonyl ($Re_2(CO)_{10}$).

In various embodiments, the metal carbonyl clusters comprise a metal selected from Group 6 or 8 of the Periodic Table of Elements. Examples of Group 6 elements include chromium, molybdenum and tungsten, and Group 8 elements include iron, ruthenium and osmium. For example, the metal carbonyl clusters may comprise or consist essentially of osmium carbonyl, molybdenum carbonyl, tungsten carbonyl, ruthenium carbonyl, or mixtures thereof.

In specific embodiments, the organometallic material comprise or consist essentially of at least one of the following compounds:

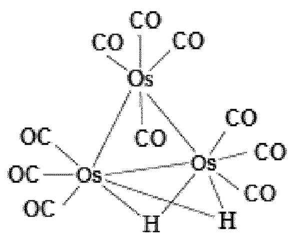 , 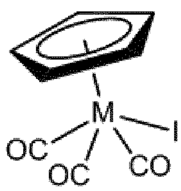 , 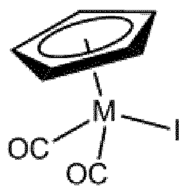 , 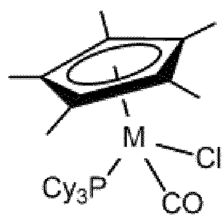 ,

or

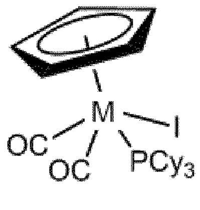 ,

wherein M = Os, Mo, W or Ru.

In some embodiments, the organometallic material comprises or consists essentially of at least one of the following compounds:

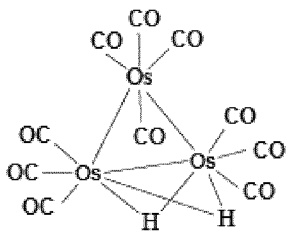

,

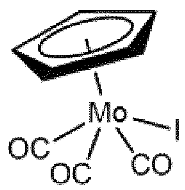

,

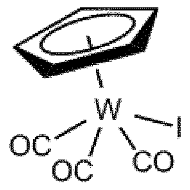

,

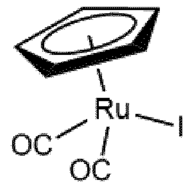

,

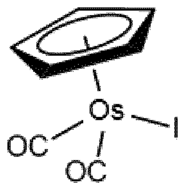

,

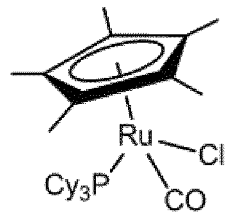

, or

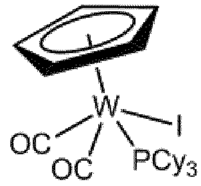

.

In one embodiment, the organometallic material consists essentially of osmium carbonyl having the formula

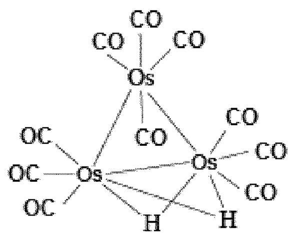

.

In this regard, the organometallic material may be considered to function as a Raman reporter, which is defined as a compound which has a high Raman cross section. Accordingly, the organometallic material may also be termed as a Raman-active marker compound, and may be considered to represent reporters of the analyte.

The organometallic material that is attached to a surface of the metallic nanoparticle may be stably adsorbed to the surface by reversible electrostatic interaction, hydrophobic interaction or covalent anchoring, to form the SERS marker conjugate. "Electrostatic attraction" relates to attachment via salt bridges, hydrogen bonds and polar interactions, for example, if the surface is charged negative and the compound bears a positive charge, and vice versa. "Hydrophobic interaction" includes the interaction between uncharged and non-polar groups. By attaching the organometallic material to the metallic nanoparticle, Raman signal from the organometallic material may be enhanced by the metallic nanoparticle.

Ideally, the organometallic material has a high Raman cross section and the capability to adsorb strongly on the surface of a metallic nanoparticle in aqueous media so that it gives a fast and intense and non-fluctuating SERS signal.

The organometallic material is attached to a surface of a metallic nanoparticle to form the SERS marker conjugate. A "nanoparticle" refers to a particle having a characteristic length, such as diameter, in the range from 1 and 100 nanometers, such as 10, 20, 30, 40, 50, 60, 70, 80, or 90 nm. The nanoparticle may be any suitable Raman enhancing nanoparticle, and may assume the form of colloidal metal, hollow or filled nanobars, magnetic, paramagnetic, conductive or insulating nanoparticles, synthetic particles, hydrogel colloids, or bars. In this regard, the organometallic material functions as a Raman active molecule, while the nanoparticle is Raman enhancing. Further, the nanoparticles may be single nanoparticles or clusters of nanoparticles.

The term "metallic nanoparticle" refers to a nanoparticle that comprises a SERS active metal. Examples of a SERS active metal include, but are not limited to, noble metals such as silver, palladium, gold, platinum, iridium, osmium, rhodium, ruthenium; copper, aluminium, or alloys thereof.

The SERS active metal may be present as a layer or coating on a nanoparticle formed from a non-SERS active material. In these embodiments, the metallic nanoparticles may have a core-shell structure, in which the core of the metallic nanoparticles is formed from any material such as plastic, ceramics, composites, glass or organic polymers, and the shell of the metallic nanoparticles is formed from a SERS active metal such as a noble metal. For example, the metallic nanoparticle may comprise a surface coating formed from a noble metal, copper, aluminium, or alloys thereof. Alternatively, the metallic nanoparticle may be formed entirely from a SERS metal, and may for example, consist of a

metal selected from the group consisting of a noble metal, copper, aluminium, and alloys thereof.

In various embodiments, the metallic nanoparticle is coated with or consists of gold, silver or alloys thereof. For example, the metallic nanoparticle may be a citrate-stabilized gold nanoparticle.

The metallic nanoparticle may be irregular or regular in shape. In some embodiments, the metallic nanoparticle is regular in shape. For example, the metallic nanoparticle may have a regular shape such as a sphere, a cube or a tetrahedron. Accordingly, the nanoparticle may be a nanosphere, a nanocube, or a nanotetrahedron.

The size of the metallic nanoparticle may be characterized by its diameter. The term "diameter" as used herein refers to the maximal length of a straight line segment passing through the center of a figure and terminating at the periphery. In embodiments where more than one metallic nanoparticle is present, size of the metallic nanoparticles may be characterized by their mean diameter, wherein the term "mean diameter" refers to an average diameter of the nanoparticles, and may be calculated by dividing the sum of the diameter of each nanoparticle by the total number of nanoparticles. Although the term "diameter" is used normally to refer to the maximal length of a line segment passing through the centre and connecting two points on the periphery of a nanosphere, it is also used herein to refer to the maximal length of a line segment passing through the centre and connecting two points on the periphery of nanoparticles having other shapes, such as a nanocube or a nanotetrahedra.

Choice of diameter of the metallic nanoparticle may depend on the type of metal that is used to coat or form the nanoparticle, which may result in differing degree of SERS signal intensity enhancement. For example, when the nanoparticle is coated with or consists of gold, the metallic nanoparticle may have a diameter of about 60 nm, which provides optimal enhancement in SERS signal intensity.

The attachment of organometallic material to metallic nanoparticles may be carried out in two ways. For example, the organometallic material may be attached to the surface of the metallic nanoparticle by metal bonding between the metallic nanoparticle and metal atom comprised in the organometallic material. For such metal bonding, the metallic nanoparticle may serve as a pi ($\pi$) donor by donating a pair of electrons to the d orbital of the metal atom comprised in the organometallic material, thereby forming metal-metal bond. This metal bonding may be strengthened by back donation of electron from organometallic materials to metallic nanoparticles through antibonding ($\pi^*$) orbital.

Additionally, the organometallic material may be attached to the metallic nanoparticle by interaction between the metallic nanoparticle and organic ligand comprised in the organometallic material. In various embodiments, the organometallic material is covalently bonded to the surface of the metallic nanoparticle. In order to facilitate covalent coupling of the organometallic material to the surface of the metallic nanoparticle, the organometallic material may include a functional group. In various embodiments, the organometallic material comprises a functional group selected from the group consisting of mercapto, carboxy, and amino. For example, the organic ligand comprised in the organometallic material may comprise a functional group selected from the group consisting of mercapto, carboxy, and amino, for attaching the organometallic material to the surface of the metallic nanoparticle.

A preferred functional group is a mercapto (-SH) group. The terms "thiol group" and "mercapto group" are used interchangeably herein and both relate to the -SH group. The mercapto group may facilitate covalent attachment to the metal surface by forming a covalent bond between the sulfur atom and a metal surface atom.

In various embodiments, the SERS marker conjugate has a diameter in the range from about 30 nm to about 100 nm. This is because SERS marker conjugates that do not fall within this size range may not have a significant SERS effect. Furthermore, SERS marker conjugates having a diameter that is less than 30 nm may be susceptible to decomposition or dissociation through interaction with the organometallic material. In various embodiments, the SERS marker conjugate has a diameter in the range from about 30 nm to 100 nm, such as in the range from about 30 nm to about 80 nm, about 30 nm to about 60 nm, about 30 nm to about 40 nm, about 40 nm to about 60 nm, about 50 nm to about 70 nm, or about 50 nm, about 60 nm, or about 70 nm. In embodiments where more than one SERS marker conjugate is present, a substantial portion or all the SERS marker conjugates may have a diameter that is in the range from about 30 nm to about 100 nm.

In various embodiments, the SERS marker conjugate further comprises a material selected from the group consisting of silica ($SiO_2$), bovineserum albumin (BSA) cross linked with glutaraldehyde, thiolated DNA, thiolated polyethylene glycol (PEG), and mixtures thereof, wherein the material is attached to the surface of the metallic nanoparticle.

In some embodiments, the material comprises or consists essentially of thiolated polyethylene glycol (PEG). In one embodiment, the material consists of thiolated polyethylene glycol (PEG). Advantageously, thiolated PEG may be used to improve stability and biocompatibility of the nanoparticles. The thiolated polyethylene glycol has a thiol functional group, which may be used to attach to the surface of a metallic nanoparticle, such as a gold nanoparticle, using the thiol functional group.

In various embodiments, the SERS marker conjugate further comprises an analyte-binding molecule coupled to the material. The term "analyte binding molecule" as used herein refers to any molecule capable of binding to an analyte of choice so as to form a complex consisting of the analyte binding molecule and the analyte.

In one embodiment of such a conjugate, the analyte binding molecule is covalently coupled to the material, which is in turn covalently attached to the nanoparticle surface. Preferably, the binding between the analyte binding

molecule to the analyte molecule is specific so that a specific complex between analyte and analyte binding molecule is formed.

"Specifically binding" and "specific binding" as used herein mean that the analyte binding molecule binds to the target analyte based on recognition of a binding region or epitope on the target molecule. The analyte binding molecule preferably recognizes and binds to the target molecule with a higher binding affinity than it binds to other compounds in the sample. In various embodiments as disclosed herein, "specifically binding" may mean that an antibody or other biological molecule, binds to a target molecule with at least about a $10^6$-fold greater affinity, preferably at least about a $10^7$-fold greater affinity, more preferably at least about a $10^8$-fold greater affinity, and most preferably at least about a $10^9$-fold greater affinity than it binds molecules unrelated to the target molecule. Typically, specific binding refers to affinities in the range of about $10^6$-fold to about $10^9$-fold greater than non-specific binding. In some embodiments, specific binding may be characterized by affinities greater than $10^9$-fold over non-specific binding. The binding affinity may be determined by any suitable method. Such methods are known in the art and include, without limitation, surface plasmon resonance and isothermal titration calorimetry. In a specific embodiment, the analyte binding molecule uniquely recognizes and binds to the target analyte.

Examples of analyte binding molecules include, but are not limited to, an antibody, antibody fragment, or antibody like molecules. In various embodiments, the analyte binding molecule is a proteinaceous molecule, such as an antibody, for example a monoclonal or polyclonal antibody, which immunologically binds to the target analyte at a specific determinant or epitope. The term "antibody" is used in the broadest sense and specifically covers monoclonal antibodies as well as antibody variants, fragments or antibody like molecules, such as for example, Fab, $F(ab')_2$, scFv, Fv diabodies and linear antibodies, so long as they exhibit the desired binding activity.

In some embodiments, the analyte binding molecule is a monoclonal antibody. The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they may be synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies can include "chimeric" antibodies and humanized antibodies. A "chimeric" antibody is a molecule in which different portions are derived from different animal species, such as those having a variable region derived from a murine mAb and a human immunoglobulin constant region.

Monoclonal antibodies may be obtained by any technique that provides for the production of antibody molecules by continuous cell lines in culture. These include, but are not limited to the hybridoma technique of Koehler and Milstein (U. S. Patent No. 4,376,110), the human B-cell hybridoma technique, and the EBV-hybridoma technique. Such antibodies may be of any immunoglobulin class including IgG, IgM, IgE, IgA, IgD and any subclass thereof. The hybridoma producing the mAb may be cultivated *in vitro* or *in vivo.* Production of high titres of mAbs *in vivo* makes this a very effective method of production.

In some embodiments as disclosed herein, the analyte binding molecule is a polyclonal antibody. "Polyclonal antibodies" refer to heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

In various embodiments, targeting ability of organometalic coated nanoparticles is achieved by incorporating a variety of analyte binding molecule. Examples of analyte binding molecules include, but are not limited to, anti-EGFR and anti-HER.

The terms "analyte", "target compound", "target molecule" or "target" as interchangeably used herein, refer to any substance that can be detected in an assay by binding to a binding molecule, and which, in one embodiment, may be present in a sample. Therefore, the analyte can be, without limitation, any substance for which there exists a naturally occurring antibody or for which an antibody can be prepared. The analyte may, for example, be an antigen, a protein, a polypeptide, a nucleic acid, a hapten, a carbohydrate, a lipid, a cell or any other of a wide variety of biological or non-biological molecules, complexes or combinations thereof. Generally, the analyte will be a protein, peptide, carbohydrate or lipid derived from a biological source such as bacterial, fungal, viral, plant or animal samples. Additionally, however, the target may also be a small organic compound such as a drug, drug-metabolite, dye or other small molecule present in the sample.

The term "sample", as used herein, refers to an aliquot of material, frequently biological matrices, an aqueous solution or an aqueous suspension derived from biological material. Samples to be assayed for the presence of an

analyte include, for example, cells, tissues, homogenates, lysates, extracts, and purified or partially purified proteins and other biological molecules and mixtures thereof.

Non-limiting examples of samples include human and animal body fluids such as whole blood, serum, plasma, cerebrospinal fluid, sputum, bronchial washing, bronchial aspirates, urine, semen, lymph fluids and various external secretions of the respiratory, intestinal and genitourinary tracts, tears, saliva, milk, white blood cells, myelomas and the like; biological fluids such as cell culture supernatants; tissue specimens which may or may not be fixed; and cell specimens which may or may not be fixed. The samples used may vary based on the assay format and the nature of the tissues, cells, extracts or other materials, especially biological materials, to be assayed. Methods for preparing protein extracts from cells or samples are well known in the art and can be readily adapted in order to obtain a sample that may be used with the SERS marker conjugates disclosed herein. Detection in a body fluid can also be *in vivo,* i.e. without first collecting a sample.

"Peptide" generally refers to a short chain of amino acids linked by peptide bonds. Typically peptides comprise amino acid chains of about 2-100, more typically about 4-50, and most commonly about 6-20 amino acids. "Polypeptide" generally refers to individual straight or branched chain sequences of amino acids that are typically longer than peptides. "Polypeptides" usually comprise at least about 20 to 1000 amino acids in length, more typically at least about 100 to 600 amino acids, and frequently at least about 200 to about 500 amino acids. Included are homo-polymers of one specific amino acid, such as for example, poly-lysine. "Proteins" include single polypeptides as well as complexes of multiple polypeptide chains, which may be the same or different.

Multiple chains in a protein may be characterized by secondary, tertiary and quaternary structure as well as the primary amino acid sequence structure, may be held together, for example, by disulfide bonds, and may include post-synthetic modifications such as, without limitation, glycosylation, phosphorylation, truncations or other processing.

Antibodies such as IgG proteins, for example, are typically comprised of four polypeptide chains (i.e., two heavy and two light chains) that are held together by disulfide bonds. Furthermore, proteins may include additional components such associated metals (e. g., iron, copper and sulfur), or other moieties. The definitions of peptides, polypeptides and proteins includes, without limitation, biologically active and inactive forms; denatured and native forms; as well as variant, modified, truncated, hybrid, and chimeric forms thereof.

The terms "contacting" or "incubating" as used interchangeably herein refer generally to providing access of one component, reagent, analyte or sample to another. For example, contacting may involve mixing a solution comprising an analyte binding protein or conjugate thereof with a sample. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The term "detecting" as used herein refers to a method of verifying the presence of a given molecule. The technique used to accomplish this is surface enhanced Raman spectroscopy (SERS). The detection may also be quantitative, i.e. include correlating the detected signal with the amount of analyte. The detection includes *in vitro* as well as *in vivo* detection.

The SERS marker conjugate as disclosed herein may also be used in a multiplex method for detecting more than one analyte, i.e. two or more different analytes. This usually requires the use of more than one analyte binding molecule in the contacting step so that each analyte is bound by a specific analyte binding molecule. The signal obtained from a multitude of different analyte binding molecule:analyte complexes may be resolved by using different Raman-active molecules or organometallic material that produce distinct SERS signals.

For example, SERS marker conjugates as disclosed herein may have a measurable SERS spectrum with a suitable excitation wavelength in the range of about 500 nm to 1000 nm. The organometallic material attached to the metallic nanoparticle serves as a reporter and may provide a detectable and unique SERS signal in 2000 $cm^{-1}$ region. As mentioned above, by providing a SERS signal in this region, this avoids interference with signals emitted by biomolecules or analytes which fall within the same region, so as to allow identification of biomolecules without the need to decouple signals emitted from the SERS marker conjugates.

These SERS marker conjugates may be part of a kit for the detection of a given analyte or the conjugate components may, together with coupling agents, form part of a kit, requiring that before use, the conjugate is formed.

In a further aspect, the disclosure relates to a biosensor for the detection of an analyte using surface-enhanced Raman spectroscopy, comprising one or more of the above conjugates. The biosensor may further comprise a substrate with the nanoparticles being attached to or adherent to the substrate. The biosensor can be configured for *in vivo* and/or *in vitro* use. The use of such a biosensor may be *in vivo* or *in vitro.*

As described above, the SERS marker conjugate or the biosensor described herein may be used in a method for the detection of an analyte, wherein the method comprises contacting the SERS marker conjugate or the biosensor with the analyte containing medium, for example a sample or body fluid, and detecting the SERS signal from the sensor. In some embodiments, the biosensor is configured for a multiplex method that allows the detection of more than one analyte.

A method of forming a surface enhanced Raman spectroscopy (SERS) marker conjugate is also disclosed herein. The method comprises mixing a suspension comprising metallic nanoparticles with a solution comprising an organometallic material to form a mixture; and incubating the mixture to allow attachment of the organometallic material to a surface of the metallic nanoparticles to form the SERS marker conjugate.

The suspension may comprise metallic nanoparticles that consist essentially of metallic nanoparticles which are coated with or consist of a metal selected from the group consisting of a noble metal, copper, aluminum, and alloys thereof. The metallic nanoparticles may be dispersed in a suitable reagent, such as ethanol, methanol, dichloromethane, chloroform, benzene, toluene, acetonitrile, tetrahydofuran, dimethyl sulfoxide, hexane, cyclohexane and mixtures thereof. Examples of noble metal that may be used to form the metallic nanoparticles have been discussed above.

The solution may comprise an organometallic material consisting essentially of metal carbonyl clusters. Examples of suitable organometallic material and metal carbonyl clusters that may be used have already been mentioned above. The organometallic material and metal carbonyl clusters may be dispersed in a suitable reagent, which may be the same as or different from that used for dispersing the metallic nanoparticles. In various embodiments, both the metallic nanoparticles and the organometallic material are dispersed in ethanol.

Incubation of the mixture may generally be carried out at mild reaction conditions such as room temperature and pressure. Incubation of the mixture may be carried out for any suitable time that allows attachment of the organometallic material to a surface of the metallic nanoparticles. In various embodiments, the incubation time is in the range from about 30 minutes to about 180 minutes, such as about 30 minutes to about 120 minutes, about 60 minutes to about 90 minutes, about 80 minutes, about 70 minutes, or about 60 minutes.

The method of forming a surface enhanced Raman spectroscopy (SERS) marker conjugate may further comprise incubating the SERS marker conjugate with a solution comprising a material selected from the group consisting of silica ($SiO_2$), bovineserum albumin (BSA) cross linked with glutaraldehyde, thiolated DNA, thiolated polyethylene glycol (PEG), and mixtures thereof, so as to attach the material to the surface of the metallic nanoparticle. In various embodiments, the solution comprises or consists essentially of thiolated polyethylene glycol (PEG), which may be added to improve stability and biocompatibility of the nanoparticles. Similar incubation time and conditions as that used to attach organometallic material to a surface of the metallic nanoparticles may be used.

In further examples, a solution comprising an analyte-binding molecule is added to the SERS marker conjugate to couple the analyte-binding molecule to the SERS marker conjugate. Examples of analyte-binding molecules which may be used have already been mentioned above.

In the afore-mentioned examples, to allow for binding of the material and/or the analyte-binding molecules to the SERS marker conjugates, an excess of the material and/or the analyte-binding molecules may be used. Accordingly, to remove the excess material and/or the analyte-binding molecules, the method of making the conjugate may also include a separation step, such as centrifugation, to separate the SERS marker conjugates formed from the excess reagents.

Hereinafter, the present invention will be described more fully with reference to the accompanying drawings, in which exemplary embodiments of the invention are shown. This invention may, however, be embodied in many different forms and should not be construed as limited to the exemplary embodiments set forth herein. Rather, these embodiments are provided so that this disclosure will be thorough and complete, and will fully convey the scope of the invention to those skilled in the art. In the drawings, lengths and sizes of layers and regions may be exaggerated for clarity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention, which is defined by the claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

## EXPERIMENTAL SECTION

Organometallic osmium carbonyl clusters, which have the advantages of being oxygen- and moisture-stable, and are very robust insofar as organometallic compounds go, have been used in the experiments as exemplary embodiments. The binding of an organometallic osmium carbonyl cluster, $Os_3(CO)_{10}(\mu\text{-}H)_2$, onto the surface of gold nanoparticles to form OM-NP constructs, has been shown to significantly enhance the inherently weak Raman signal of the metal carbonyl CO stretching vibrations.

As demonstrated, the Raman spectrum of the osmium carbonyl cluster or OM-NP constructs shows no detectable CO signal in an ethanol:water (1:4, v/v) solution at 10 $\mu$M concentration; it can only be detected at a much higher concentration (50 mM). In contrast thereto, the CO signals in the OM-NP constructs are significantly enhanced, wherein the CO intensity has increased by over four orders of magnitude (a factor of about 15000).

### Example 1: General Procedure

All manipulations for chemical synthesis were carried out using standard Schlenk techniques under an argon or nitrogen atmosphere. The triosmium carbonyl cluster, $Os_3(CO)_{10}(\mu\text{-}H)_2$, was prepared according to procedure reported in H. D. Kaesz, Inorg. Synth. 1990, 28, 238-240. $Os_3(CO)_{12}$ was purchased from Oxkem; all other chemicals were purchased from other commercial sources and used as supplied.

UV-vis spectra were recorded using a Beckman Coulter DU 730 spectrometer. Transmission electron microscopy (TEM) images were recorded on a JEOL JEM 3010 TEM at an accelerating voltage of 300 kV. TEM samples were prepared by placing a drop of the nanoparticles onto a carbon coated Cu grid.

Mean particle sizes were obtained by measuring the sizes of the nanoparticles in a few randomly chosen areas of the digitized image, each containing approximately 100 to 200 nanoparticles.

IR spectra were obtained using a Bruker Alpha Fourier transform infrared spectrometer. The spectral measurements were carried out using a Renishaw In Via Raman (UK) microscope with a Peltier cooled CCD detector and an excitation wavelength at 633 nm, where the laser beam is directed to the sample through a 50 $\times$ objective lens, which was used to excite the sample and also to collect the return Raman signal. Raman spectrometers by other manufacturers are also suitable for use with SERs marker conjugates of the present disclosure. All Raman spectra were processed with WiRE3.0 software. The maximum laser power at the sample was measured to be 6.2 mW and the exposure time was set at 10 s throughout the measurements. Prior to each measurement, the instrument was calibrated with a silicon standard whose Raman peak is centered at 520 $cm^{-1}$.

### Example 2: Preparation of OM-NP constructs and OM-NP(PEG)-L constructs

Freshly prepared $Os_3(CO)_{10}(\mu\text{-}H)_2$ ethanol solutions of various concentrations (10 $\mu$M, 20 $\mu$M, 60 $\mu$M, 80 $\mu$M, and 100 $\mu$M) were mixed with 60 nm gold colloid (2.6 $\times$ $10^{10}$ particles/mL, BBInternational UK) in ethanol to form OM-NP constructs. The optimal molar concentration of $Os_3(CO)_{10}(\mu\text{-}H)_2$ was found to be 100 $\mu$M.

After incubating for 60 min, excess $Os_3(CO)_{10}(\mu\text{-}H)_2$ was removed by centrifugation (10,000 rpm, 2 min), and the OM-NP constructs were re-suspended in 1 mL deionized (DI) water for subsequence bioconjugation. The OM-NP constructs were incubated with 10 $\mu$M of thiolated polyethylene glycol (PEG) (HS-PEG-COOH, $M.W._{PEG}$ 5000 Da, RAPP Polymere GmbH) for 60 min to form peglyated OM-NP constructs. Excess HS-PEG-COOH was removed by centrifugation (10,000 rpm, 2 min), and the peglyated OM-NP constructs were re-suspended in 1 mL DI water, where the carboxyl terminal of PEG is ready for conjugation with an antibody. The peglyated OM-NP constructs were incubated with ethyl dimethylaminopropyl (Sigma-Aldrich) and N-hydroxy succimide (Sigma-Aldrich) at 25 $\mu$M each respectively, and 100 $\mu$L (100 ng/ mL) anti-EGFR $IgG_{2a}$ (Santa Cruz) was then added to form OM-NP(PEG)-L constructs.

After overnight incubation, excess of anti-EGFR, ethyl dimethylaminopropyl (EDC) and N-hydroxy succimide were removed by centrifugation (10,000 rpm, 2 min) Thiolated PEG (HS-PEG, $M.W._{PEG}$ 5000 Da, RAPP Polymere

GmbH) was added to achieve maximum encapsulation with PEG. After incubating for 30 min, excess HS-PEG was removed by centrifugation (10,000 rpm, 2 min), and the OM-NP(PEG)-L constructs were suspended in DI water for storage.

**Example 3: SERS signal stability of OM-NP and OM-NP(PEG)-L constructs**

The SERS signal of the OM-NP constructs was measured over a period of a month. SERS spectra were obtained upon excitation with a 633 nm laser (60 mW power), and the SERS intensity of the highest Raman peak (2030 $cm^{-1}$) was plotted as mean ± standard deviation of three independent measurements taken from the same sample at different times.

**FIG. 3(A)** shows that there is a detectable Raman scattering signal from Os-Au nanoparticles in aqueous solution. These OM-NP constructs also exhibit remarkably good storage stability. **FIG. 4** shows that there is consistent Raman scattering signal in water up to 28 days. The CO signals in aqueous solution remain consistent up to 28 days as can be seen from **FIG. 4A and B,** since any decomposition of the metal carbonyl on the gold nanoparticle surface would have shown up as a change in the CO signal intensity. Os-Au nanoparticles or OM-NP constructs exhibit good stability to bioconjugation process with minimal Raman scattering signal decrease, ca. 10 %. As the transmission electron microscope (TEM) images in **FIG. 4C and D** show, the constructs are well dispersed in the aqueous solution, and show no aggregation even after 30 days. The constructs are spherical, with an average size of 60 nm.

**FIG. 6** are graphs showing time course study and spectra of (A) OM-NP constructs, and (B) OM-NP(PEG)-L constructs collected over 28 days in water.

**Example 4: Cell viability study for OM-NP constructs**

For the live cell imaging, the OM-NP constructs were conjugated with an antibody against epidermal growth factor receptors (anti-EGFR), as this is highly expressed in diverse cancers and hence have been used in many biological studies. Successful conjugation was confirmed by the observation of an absorbance maximum at 280 nm as shown in **FIG. 5.** PEG was also added in the bioconjugation process to improve the stability and biocompatibility of the constructs. These OM-NP(PEG)-L constructs also exhibited good storage stability, with minimal detriment (< 10 %) in the CO signal over 28 days as shown in **FIG. 4(B).**

Five thousand OSCC cells were seeded in a 96-well plate for 24 hours before introducing each well with EGFR-PEG Os-Au nanoparticles (OM-NP(PEG)-L constructs) (43 pM). The OSCC cells were allowed to incubate for another 24 hours, after which 10 μl of CCK8 (cell counting proliferation kit, Sigma-Aldrich) was added to each well. Cell absorbance was measured with a SpectraMax 384 Plus spectral analyser after 4 hours at 450 nm excitation.

The cytotoxicity of these OM-NP(PEG)-L constructs were assessed with the OSCC cell line (epidermoid carcinoma), in which EGFR is frequently overexpressed. Interestingly, while the cluster $Os_3(CO)_{10}(\mu\text{-}H)_2$ is clearly cytotoxic, the constructs are not, as the cells remained about 100 % viable with respect to the control as can be seen in **FIG. 7.** This shows that Os-Au nanoparticles or OM-NP constructs are safe for use in biological studies.

**Example 5: SERS mapping experiments in OSCC and SKOV3 cells.**

SERS mapping experiments were performed in a Renishaw InVia Raman microscope system with a laser beam directed to the sample through a 50 × objective lens, and a Peltier cooled CCD detector. OSCC (epidermoid carcinoma) and SKOV3 (ovarian carcinoma) cells were plated in an 8-well glass slide at a density of $10^6$ cells/mL, in Dulbecco Modified Eagle's Medium (DMEM, Gibco) containing 10 % fetal bovine serum and penicillin streptomycin (Gibco). All cultures were maintained at 37 °C with 5 % carbon dioxide ($CO_2$).

After incubation with EGFR-PEG Os-Au nanoparticles (43 pM) for 4 h at 25 °C rinsed with PBS (× 3) and media (× 2, 15 min incubation per wash), samples were excited with a 633 nm excitation wavelength with a laser focal spot of 1 μm and 6 mW power and mapping measurements at 2030 $cm^{-1}$ were carried out as raster scans in 1 μm steps over the specified area (aprox. 30 x 30 $\mu m^2$) with 1 s as the integration time per step.

The cells were subsequently mounted with Vectasheild fluorescent mounting medium for dark-field microscopy experiments. Cells were visualized using an enhanced dark field (EDF) illumination system (CytoViva) attached to a Nikon Eclipse 80i microscope. The system consisted of a CytoViva 150 dark-field condenser, that was in place of the original condenser of the microscope, and attached via a fiber optic light guide to a Solarc 24 W metal halide light source. Images were taken under a 100 × oil objective lens with an iris.

Two different cancer cell lines, namely, OSCC (epidermoid carcinoma, EGFR-positive) and SKOV3 (ovarian carcinoma, EGFR-negative) cells were employed to confirm the specificity of the OM-NP(PEG)-L constructs.

OSCC cells that have been treated with the OM-NP(PEG)-L constructs are imaged using the SERS-enhanced CO absorption peak at 2,030 $cm^{-}1$, and the image is closely correlated with the bright-field and dark-field imaging results

in **FIG. 8(A) to (D).**

A similar set of images obtained with an EGFR-negative cell line, SKOV3 (ovarian carcinoma), is also shown in **FIG. 8(E) to (H),** which clearly shows the absence of the constructs. This study clearly shows the advantages of OM-NP constructs for live cell imaging, for which the OM-NP constructs signal is separated from cell molecules signals.

A dark-field microscope was used for OM-NP(PEG)-L constructs visualization in cells. A magnified dark-field image of one each of these two treated cell lines clearly show that there is much stronger light scattering from the OSCC cells compared to the SKOV3 cells **(FIG. 8(B) and (F)**). This clearly shows the specificity and efficient targeting of OM-NP(PEG)-L constructs to EGFR positive cells.

The OSCC cells were then stored for 3 days to examine the stability of OM-NP(PEG)-L constructs in cellular system. Results are shown in **FIG. 9.** Comparing to the original signal, no changes were observed for CO signal of OM-NP(PEG)-L constructs in term of peak shifting. Further, the cells still afford strong images after having been stored for 3 days. This indicates that OM-NP(PEG)-L constructs are stable in the cellular environment.

**FIG. 10** shows that the Raman scattering signal of osmium carbonyl clusters was observed from OSCC cells. The scattering bright spots are clearly correlated with the locations at which the CO vibration signals can be found.

## Example 6: Estimation of CO peak enhancement

SERS has been used herein to enhance CO stretching vibration signal of metal carbonyl compounds. The enhancement of the CO vibration peak intensity was estimated by comparing the intensity of the 2030 $cm^{-1}$ peak for the cluster $Os_3(CO)_{10}(\mu\text{-}H)_2$ and the OM-NP constructs as:

$$\text{Enhancement} = (C_{cluster} \times I_{construct}) / (C_{construct} \times I_{cluster})$$

where $C_{cluster}$ and $C_{construct}$ are the concentration, and $I_{cluster}$ and $I_{construct}$ the corresponding normal Raman and SERS intensities, for the cluster $Os_3(CO)_{10}(\mu\text{-}H)_2$ and the OM-NP constructs respectively.

The extinction coefficient ($\varepsilon$) of the 2025 $cm^{-1}$ peak of $Os_3(CO)_{10}(\mu\text{-}H)_2$ in ethanol was determined to be 8850 $M^{-1}$ $cm^{-1}$; an IR spectrum (absorbance mode) is given in **FIG. 11,** and the Beer's Law plot in **FIG. 12.**

The concentration of clusters in the OM-NP construct was estimated as follows:

A 10 ml sample of a 4.3 x $10^{-5}$ $\mu$M suspension of gold nanoparticle (gold nanoparticles concentration was obtained from BBInternational UK product data sheet) was pelleted by centrifugation. This was dispersed in 1.0 ml of a 1000 $\mu$M solution of $Os_3(CO)_{10}(\mu\text{-}H)_2$ in ethanol, incubated for 30 min and then centrifuged again. An aliquot of the supernatant was diluted 6 x and the concentration of unreacted cluster was determined by measuring the IR absorbance at 2025 $cm^{-1}$. The concentration of cluster in the OM-NP constructs was then estimated, based on the difference in these cluster concentrations, to be 340 $\mu$M.

For the Raman measurements, the constructs were first prepared as described above but with a 10 x dilution, so that the concentration of cluster in the OM-NP constructs is about 34 $\mu$M. These were then pelleted by centrifugation and then resuspended in the same volume of water. The intensity of the 2030 $cm^{-1}$ peak in the Raman spectrum of a 50 mM solution of $Os_3(CO)_{10}(\mu\text{-}H)_2$ in ethanol:water (1:4, v/v) **(FIG. 13),** and of the OM-NP constructs **(FIG. 14),** were measured at 442 counts and 4577 counts respectively. From this, enhancement of the CO signal is estimated to be: (50 mM x 4577) / (34 $\mu$M x 442) = 15228.

## Example 7: Preparation of oraganometallic (Molybdenum, Osmium, Ruthenium, Tungsten) carbonyl coated nanoparticles as SERS nanotags

The half sandwich complexes **1 - 4** as shown in **FIG. 15** have been synthesized and conjugated to gold nanoparticles to give SERS signal in the region of 2200-1800 $cm^{-1}$. A freshly prepared organometallic carbonyl ethanol solution with various concentrations (20 $\mu$ M) was mixed with gold colloid (2.6 $\times$ $10^{10}$ particles/mL, BBInternational UK) in ethanol. After 60 min incubation, the excess of organometallic carbonyl was removed by centrifugation (10000 rpm, 2 min), and organometallic carbonyl-Au pellet was resuspended in 1 mL DI water for SERS measurement.

As mentioned above, state of the art SERS reporter molecules emit signals in the region of 800 $cm^{-1}$ to 1800 $cm^{-1}$ under SERS, which is unfortunately, a region where much signals of biomolecules is emitted, thereby resulting in peak overlapping between those of SERS reporters and analytes.

By using a SERS marker conjugate comprising a metallic nanoparticle and an organometallic material attached to a surface of the metallic nanoparticle disclosed herein, the above problem may be addressed. Embodiments of the SERS marker conjugate include an organometallic metal carbonyl-based biotag, which is formed by attaching osmium carbonyl clusters to gold nanoparticles, as an example of a OM-NP construct. Strong SERS carbonyl signal in 2000 $cm^{-1}$ region was observed for OM-NP constructs in aqueous solution. By using transition metal carbonyl compounds,

for which the CO stretching vibration signal is well-separated from other molecular vibrational modes of the cell, signals from live cell imaging may be identified readily. Furthermore, when compared to other detection modes, the SERS marker conjugates disclosed herein offers improved sensitivity in terms of signal strength and signal-to-noise ratio, as well as good spatial resolution.

Furthermore, these OM-NP constructs display excellent aqueous dispersibility and storage stability. The OM-NP constructs may be readily functionalized with suitable binding ligands to produce biologically functional OM-NP(PEG)-L constructs, as demonstrated in live cells imaging experiments detailed above. For example, the SERS nanotags may be subsequently coated with polymers such as polyethylene glycol (M.W. 5000 Da, PEG) to increase their storage stability, and to increase their retention time for possible *in-vivo* applications. Specificity of the SERS nanotags may be optimized by incorporating targeting molecules such as antibodies and DNA. Ease of bio-functionalization, good biocompatibility with biomolecules, high stability and good dispersibility in aqueous solution, means that the SERS marker conjugates disclosed herein form excellent candidates for biomedical applications, such as multiplexed biological assay, immunoassay, chemical assay, and other tests known in the biological, chemical forensic, genomic, and medical areas.

## Claims

1. Method for detecting one or more analytes by surface enhanced Raman spectroscopy (SERS) using a SERS marker conjugate comprising a metallic nanoparticle and an organometallic material comprising or consisting essentially of a metal carbonyl compound, the organometallic material being attached to a surface of the metallic nanoparticle by metal bonding between the metallic nanoparticle and metal atom comprised in the organometallic material,
the method comprising

    a) contacting the one or more analytes with at least one analyte binding molecule attached to the SERS marker conjugate; and
    b) detecting a surface enhanced Raman signal from the SERS marker conjugate.

2. Method according to claim 1, wherein the metal carbonyl compound comprises or consists essentially of metal carbonyl clusters.

3. Method according to claim 2, wherein the metal carbonyl clusters (i) comprise a metal selected from Group 6 or 8 of the Periodic Table of Elements, or (ii) comprise or consist essentially of osmium carbonyl, molybdenum carbonyl, tungsten carbonyl, ruthenium carbonyl, or mixtures thereof.

4. Method according to claim 1, wherein the organometallic material comprises or consists essentially of at least one of the following compounds:

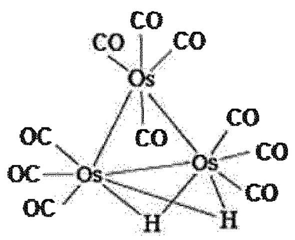 , 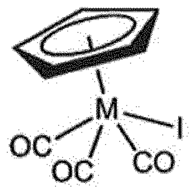 , 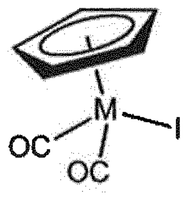 , 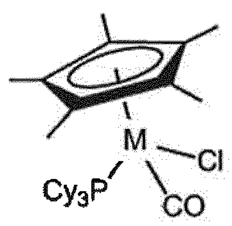 ,

or

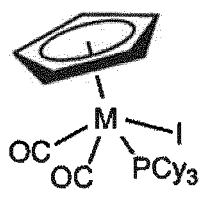 ,

wherein M = Os, Mo, W or Ru.

**5.** Method according to claim 1, wherein the organometallic material comprises or consists essentially of at least one of the following compounds:

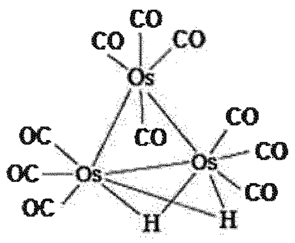
,
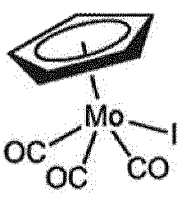
,
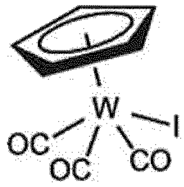
,
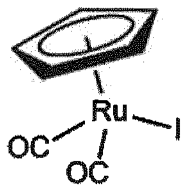
,
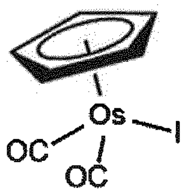
,
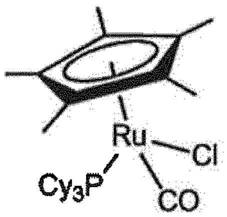
, or
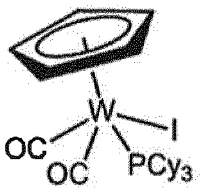
.

**6.** Method according to claim 1, wherein the metallic nanoparticle is coated with or consists of (i) a metal selected from the group consisting of a noble metal, copper, aluminum, and alloys thereof, or (ii) gold, silver, or alloys thereof.

**7.** Method according to claim 1, wherein the organometallic material is additionally attached to the metallic nanoparticle by interaction between the metallic nanoparticle and organic ligand comprised in the organometallic material.

**8.** Method according to claim 7, wherein the organic ligand comprised in the organometallic material comprises a functional group selected from the group consisting of mercapto, carboxy, and amino, for attaching the organometallic material to the surface of the metallic nanoparticle.

**9.** Method according to claim 1, further comprising a material selected from the group consisting of silica ($SiO_2$), bovineserum albumin (BSA) cross linked with glutaraldehyde, thiolated DNA, and thiolated polyethylene glycol (PEG), and mixtures thereof, wherein the material is attached to the surface of the metallic nanoparticle.

**10.** Method according to claim 9, wherein the material comprises or consists essentially of thiolated polyethylene glycol (PEG).

**11.** Method according to claim 9, further comprising an analyte-binding molecule coupled to the material.

**12.** Method according to claim 11, wherein the analyte binding molecule is selected from the group consisting of an antibody, antibody fragment or antibody like molecules.

**13.** Method according to claim 1, wherein the SERS marker conjugate has a diameter in the range from about 30 nm to about 100 nm.

**14.** Method according to claim 1, wherein the SERS marker conjugate is adapted to provide a SERS signal in the region of 1800 $cm^{-1}$ to 2200 $cm^{-1}$.

**15.** A biosensor for the detection of an analyte, the biosensor comprising a plurality of SERS marker conjugates, each SERS marker conjugate comprising a metallic nanoparticle and an organometallic material comprising or consisting essentially of a metal carbonyl compound, wherein the organometallic material is attached to a surface of the metallic nanoparticle by metal bonding between the metallic nanoparticle and metal atom comprised in the organometallic material, wherein the biosensor further comprises a substrate with the nanoparticle being attached to or adherent to the substrate.

**Patentansprüche**

1. Verfahren zum Bestimmen eines oder mehrerer Analyten durch oberflächenverstärkte Raman Spektroskopie (SERS), unter Verwenden eines SERS-Markerkonjugats, das einen metallischen Nanopartikel und ein organometallisches Material, umfassend oder bestehend aus im Wesentlichen einer Metallcarbonylverbindung umfasst, wobei das organometallische Material an eine Oberfläche des metallischen Nanopartikels durch Metallbindung zwischen dem metallischen Nanopartikel und dem Metallatom, das in dem organometallischen Material enthalten ist, gebunden ist,
wobei das Verfahren umfasst:

   a) In Kontakt bringen des einen oder mehrerer Analyten mit mindestens einem Analyt-bindenden Molekül, das an das SERS-Markerkonjugat gebunden ist; und
   b) Nachweisen eines oberflächenverstärkten Ramansignals des SERS-Makerkonjugats.

2. Verfahren gemäß Anspruch 1, wobei die Metallcarbonylverbindung Metallcarbonylcluster umfasst oder im Wesentlichen daraus besteht.

3. Verfahren gemäß Anspruch 2, wobei die Metallcarbonylcluster (i) ein Metall, ausgewählt aus der Gruppe 6 oder 8 des Periodensystems der Elemente, umfassen oder (ii) Osmiumcarbonyl, Molybdäncarbonyl, Wolframcarbonyl, Rutheniumcarbonyl oder Mischungen davon umfassen oder im Wesentlich daraus bestehen.

4. Verfahren gemäß Anspruch 1, wobei das organometallische Material mindestens eine der folgenden Verbindungen umfasst oder im Wesentlichen daraus besteht:

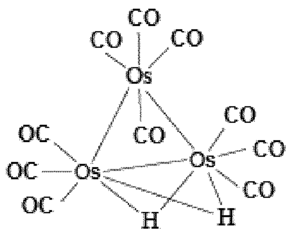
,
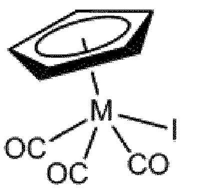
,
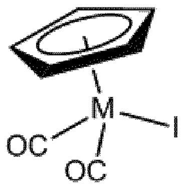
,
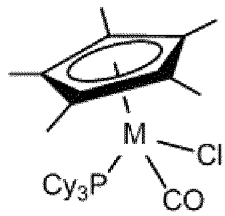
,

oder

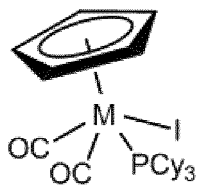

wobei M = Os, Mo, W oder Ru ist.

5. Verfahren gemäß Anspruch 1, wobei das organometallische Material mindestens eine der folgenden Verbindungen umfasst oder im Wesentlichen daraus besteht:

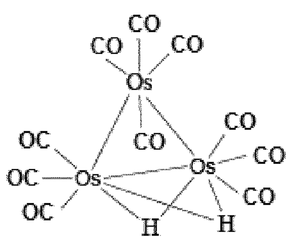
,
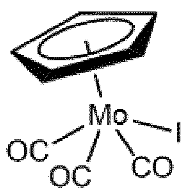
,
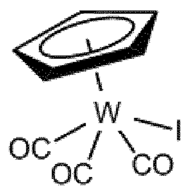
,
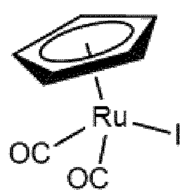
,

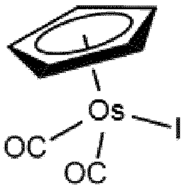, 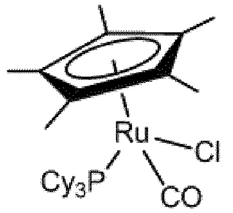, oder 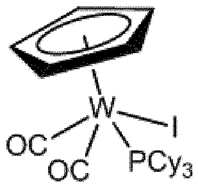.

**6.** Verfahren gemäß Anspruch 1, wobei der metallische Nanopartikel beschichtet ist oder besteht aus (i) einem Metall ausgewählt aus der Gruppe bestehend aus einem Edelmetall, Kupfer, Aluminium, und Legierungen davon, oder (ii) Gold, Silber oder Legierungen davon.

**7.** Verfahren gemäß Anspruch 1, wobei das organometallische Material zusätzlich an den metallischen Nanopartikel, durch Interaktion zwischen dem metallischen Nanopartikel und dem organischen Liganden, der in dem organometallischen Material enthalten ist, gebunden ist.

**8.** Verfahren gemäß Anspruch 7, wobei der organische Ligand, der in dem organometallischen Material enthalten ist, eine funktionelle Gruppe umfasst, ausgewählt aus der Gruppe bestehend aus Mercapto, Carboxy und Amino, zum Binden des organometallischen Materials an die Oberfläche des metallischen Nanopartikels.

**9.** Verfahren gemäß Anspruch 1, ferner umfassend ein Material ausgewählt aus der Gruppe bestehend aus Siliziumdioxid ($SiO_2$), Rinderserumalbumin (BSA) vernetzt mit Glutaraldehyd, thiolierter DNA und thioliertem Polyethylenglykol (PEG) und Mischungen davon, wobei das Material an die Oberfläche des metallischen Nanopartikels gebunden ist.

**10.** Verfahren gemäß Anspruch 9, wobei das Material thioliertes Polyethylenglykol (PEG) umfasst oder im Wesentlichen daraus besteht.

**11.** Verfahren gemäß Anspruch 9, ferner umfassend ein Analyt-bindendes Molekül, das an das Material gekoppelt ist.

**12.** Verfahren gemäß Anspruch 11, wobei das Analyt-bindende Molekül ausgewählt ist aus der Gruppe bestehend aus einem Antikörper, Antikörperfragment oder Antikörper-ähnlichen Molekülen.

**13.** Verfahren gemäß Anspruch 1, wobei das SERS-Markerkonjugat einen Durchmesser im Bereich von etwa 30 nm bis etwa 100 nm aufweist.

**14.** Verfahren gemäß Anspruch 1, wobei das SERS-Markerkonjugat so adaptiert ist, um ein SERS-Signal im Bereich von 1800 $cm^{-1}$ bis 2200 $cm^{-1}$ bereitzustellen.

**15.** Ein Biosensor für den Nachweis eines Analyten, wobei der Biosensor eine Vielzahl von SERS-Markerkonjugaten umfasst, wobei jedes SERS-Markerkonjugat einen metallischen Nanopartikel und ein organometallisches Material, das eine Metallcarbonylverbindung umfasst oder im Wesentlichen daraus besteht, umfasst, wobei das organometallische Material an eine Oberfläche des metallischen Nanopartikels durch Metallbindung zwischen dem metallischen Nanopartikel und dem Metallatom, das in dem organometallischen Material enthalten ist, gebunden ist, wobei der Biosensor ferner ein Substrat mit einem Nanopartikel, der an das Substrat gebunden ist oder daran anhaftet, umfasst.

## Revendications

**1.** Procédé de détection d'un ou plusieurs analytes par spectroscopie Raman exaltée de surface (SERS) à l'aide d'un conjugué de marqueur de SERS comprenant une nanoparticule métallique et un matériau organométallique comprenant un composé métal-carbonyle ou essentiellement constitué d'un composé métal-carbonyle, le matériau organométallique étant fixé à une surface de la nanoparticule métallique par liaison métallique entre la nanoparticule métallique et l'atome de métal compris dans le matériau organométallique,
le procédé comprenant

a) la mise en contact des un ou plusieurs analytes avec au moins une molécule se liant aux analytes fixée au conjugué de marqueur de SERS ; et

b) la détection d'un signal de spectroscopie Raman exaltée de surface provenant du conjugué de marqueur de SERS.

**2.** Procédé selon la revendication 1, dans lequel le composé métal-carbonyle comprend des agrégats métal-carbonyle ou est essentiellement constitué d'agrégats métal-carbonyle.

**3.** Procédé selon la revendication 2, dans lequel les agrégats métal-carbonyle (i) comprennent un métal choisi dans le groupe 6 ou 8 du tableau périodique des éléments, ou (ii) comprennent de l'osmium-carbonyle, du molybdène-carbonyle, du tungstène-carbonyle, du ruthénium-carbonyle ou des mélanges de ceux-ci, ou sont essentiellement constitués de ceux-ci.

**4.** Procédé selon la revendication 1, dans lequel le matériau organométallique comprend au moins l'un des composés suivants ou est essentiellement constitué d'au moins l'un de ceux-ci :

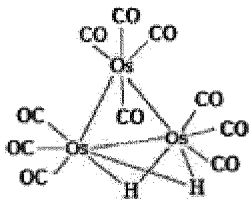 , 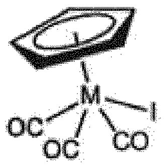 , 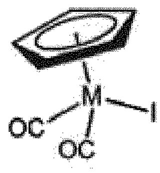 , 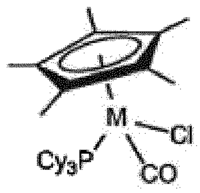

ou

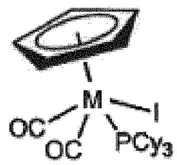 ,

formules dans lesquelles M = Os, Mo, W ou Ru.

**5.** Procédé selon la revendication 1, dans lequel le matériau organométallique comprend au moins l'un des composés suivants ou est essentiellement constitué d'au moins l'un de ceux-ci :

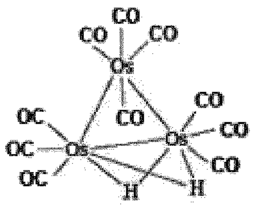 , 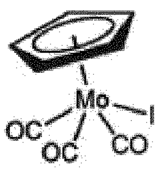 , 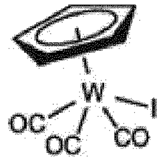 , 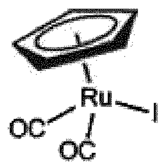 ,

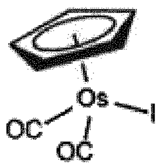 , 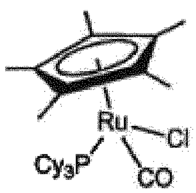 ou 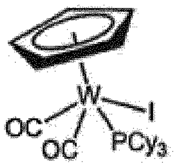 .

**6.** Procédé selon la revendication 1, dans lequel la nanoparticule métallique est revêtue ou est constituée de (i) un

métal choisi dans le groupe constitué d'un métal noble, du cuivre, de l'aluminium et des alliages de ceux-ci, ou (ii) d'or, d'argent ou d'alliages de ceux-ci.

**7.** Procédé selon la revendication 1, dans lequel le matériau organométallique est en outre fixé à la nanoparticule métallique par interaction entre la nanoparticule métallique et le ligand organique compris dans le matériau organométallique.

**8.** Procédé selon la revendication 7, dans lequel le ligand organique compris dans le matériau organométallique comprend un groupe fonctionnel choisi dans le groupe constitué des groupes mercapto, carboxy et amino, pour fixer le matériau organométallique à la surface de la nanoparticule métallique.

**9.** Procédé selon la revendication 1, comprenant en outre un matériau choisi dans le groupe constitué de silice ($SiO_2$), de sérum-albumine bovin (BSA) réticulé avec du glutaraldéhyde, d'ADN thiolé et de polyéthylène glycol (PEG) thiolé, et des mélanges de ceux-ci, dans lequel le matériau est fixé à la surface de la nanoparticule métallique.

**10.** Procédé selon la revendication 9, dans lequel le matériau comprend du polyéthylène glycol (PEG) thiolé ou est essentiellement constitué de celui-ci.

**11.** Procédé selon la revendication 9, comprenant en outre une molécule se liant aux analytes couplée au matériau.

**12.** Procédé selon la revendication 11, dans lequel la molécule se liant aux analytes est choisie dans le groupe constitué d'un anticorps, d'un fragment d'anticorps ou de molécules analogues à des anticorps.

**13.** Procédé selon la revendication 1, dans lequel le conjugué de marqueur de SERS a un diamètre dans la plage d'environ 30 nm à environ 100 nm.

**14.** Procédé selon la revendication 1, dans lequel le conjugué de marqueur de SERS est adapté pour fournir un signal de SERS dans la région de 1 800 $cm^{-1}$ à 2 200 $cm^{-1}$.

**15.** Biocapteur pour la détection d'un analyte, le biocapteur comprenant une pluralité de conjugués de marqueur de SERS, chaque conjugué de marqueur de SERS comprenant une nanoparticule métallique et un matériau organométallique comprenant un composé métal-carbonyle ou essentiellement constitué d'un composé métal-carbonyle, dans lequel le matériau organométallique est fixé à une surface de la nanoparticule métallique par liaison métallique entre la nanoparticule métallique et l'atome de métal compris dans le matériau organométallique, dans lequel le biocapteur comprend en outre un substrat avec la nanoparticule étant fixée ou collée au substrat ou adhérant à celui-ci.

FIG. 1

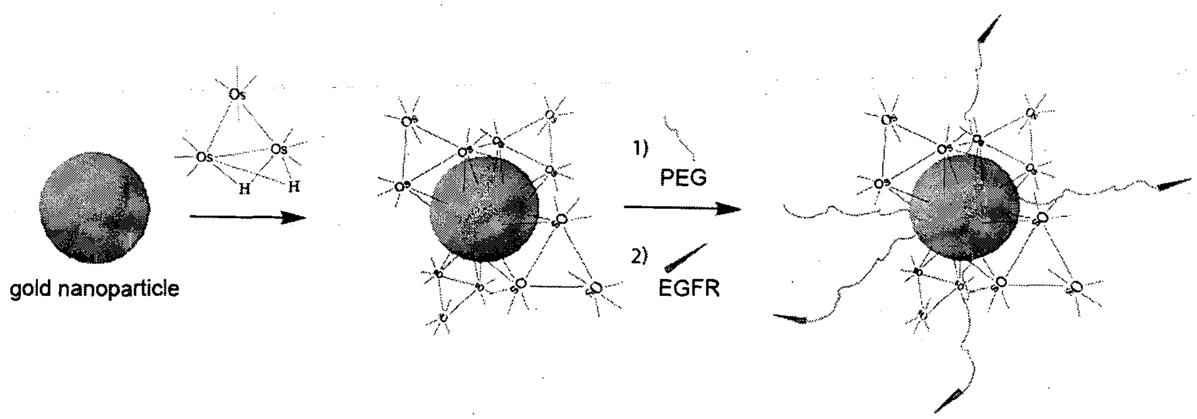
gold nanoparticle
1) PEG
2) EGFR

FIG. 2

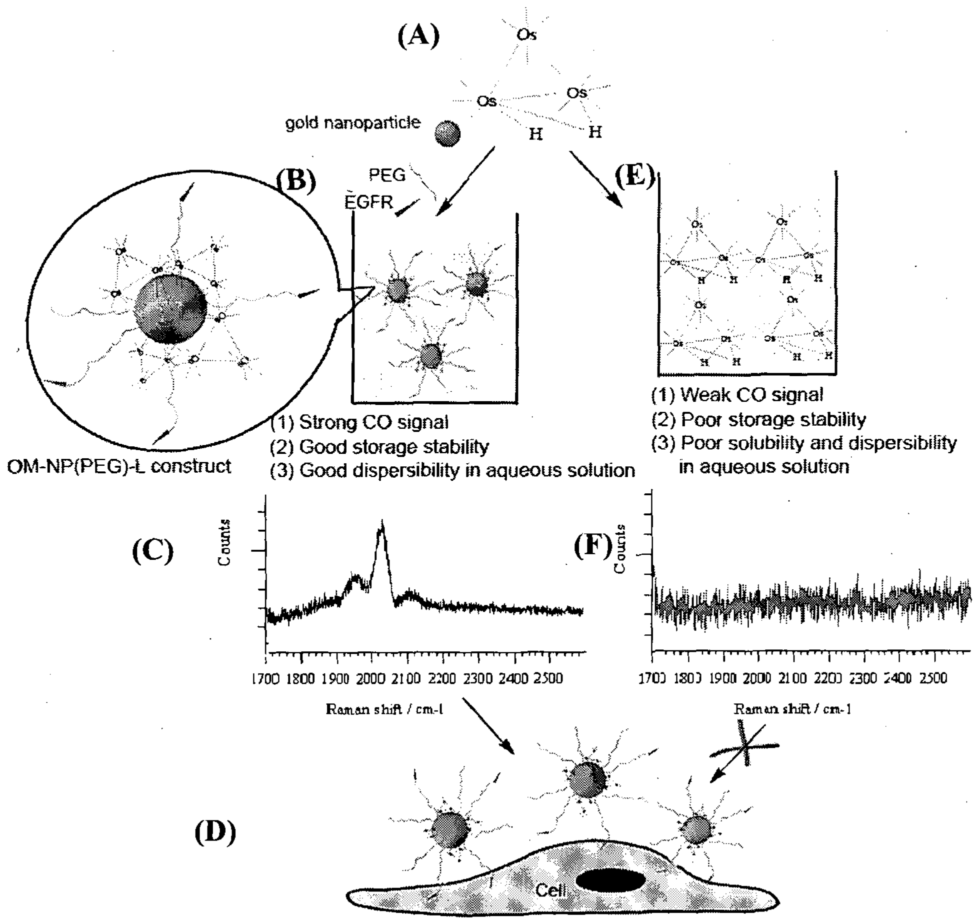
(A)
gold nanoparticle
(B)
PEG
EGFR
(E)
OM-NP(PEG)-L construct
(1) Strong CO signal
(2) Good storage stability
(3) Good dispersibility in aqueous solution
(1) Weak CO signal
(2) Poor storage stability
(3) Poor solubility and dispersibility in aqueous solution
(C)
Counts
1700 1800 1900 2000 2100 2200 2300 2400 2500
Raman shift / cm-1
(F)
Counts
1700 1800 1900 2000 2100 2200 2300 2400 2500
Raman shift / cm-1
(D)
Cell

FIG. 3

(A)

(B)

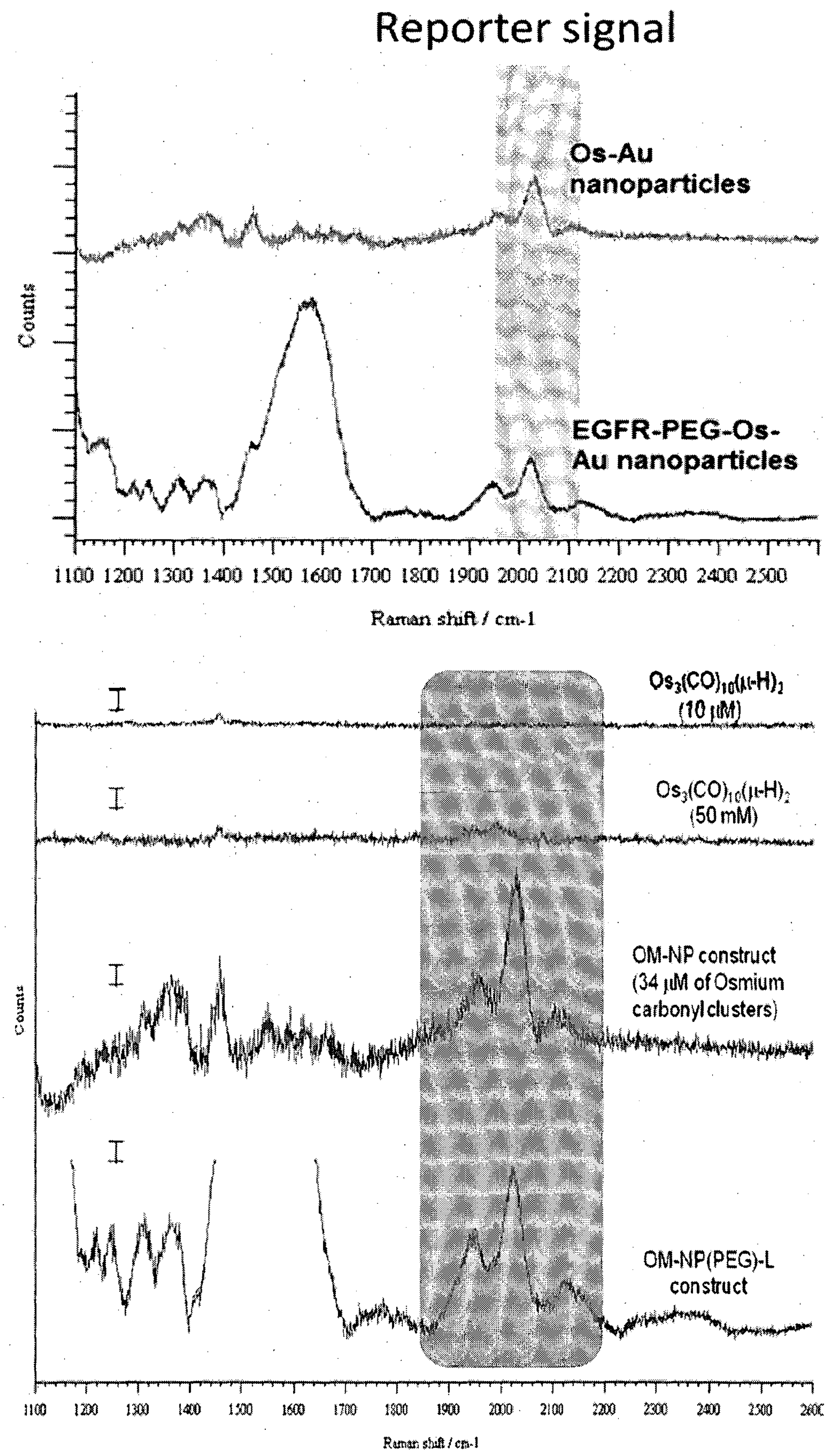
Reporter signal
Os-Au nanoparticles
EGFR-PEG-Os-Au nanoparticles
Counts
1100 1200 1300 1400 1500 1600 1700 1800 1900 2000 2100 2200 2300 2400 2500
Raman shift / cm-1
$Os_3(CO)_{10}(\mu\text{-}H)_2$ (10 μM)
$Os_3(CO)_{10}(\mu\text{-}H)_2$ (50 mM)
OM-NP construct (34 μM of Osmium carbonyl clusters)
OM-NP(PEG)-L construct
Counts
1100 1200 1300 1400 1500 1600 1700 1800 1900 2000 2100 2200 2300 2400 2500 2600
Raman shift / cm-1

FIG. 4

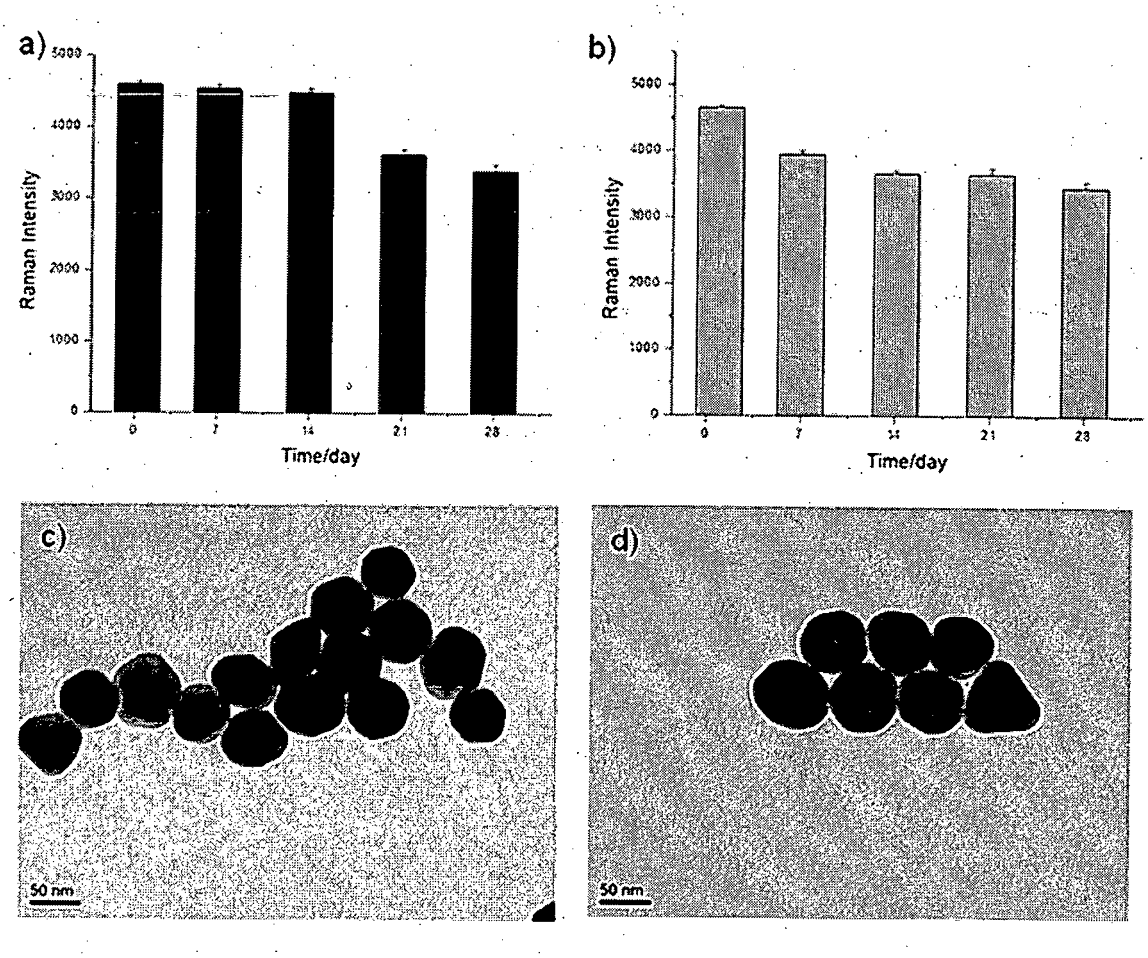
a)
b)
Raman Intensity
Time/day
0
7
14
21
28
c)
d)
50 nm

FIG. 5

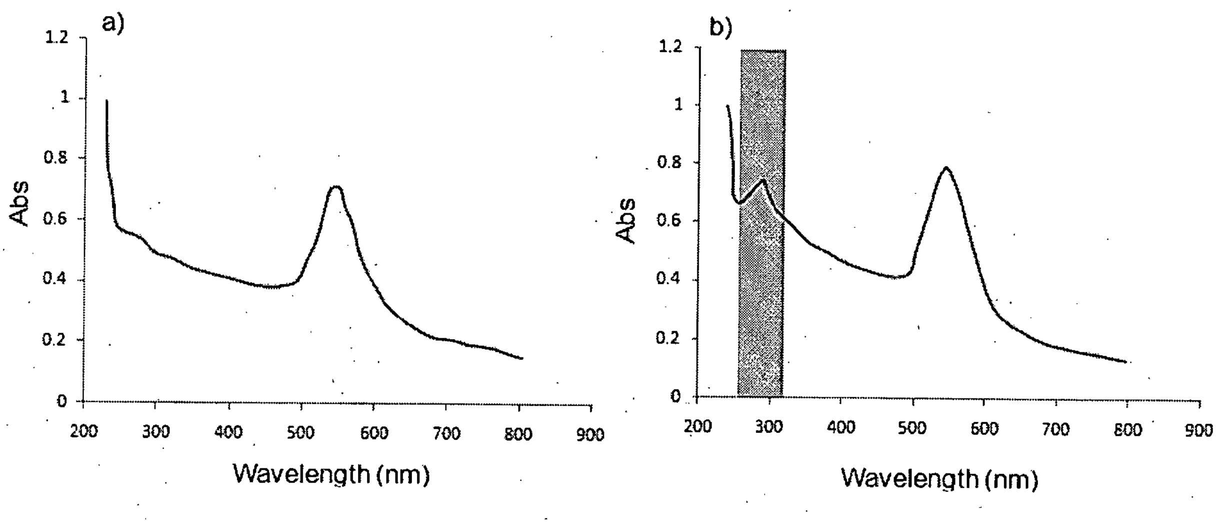
a)
b)
Abs
Wavelength (nm)
200
300
400
500
600
700
800
900
0
0.2
0.4
0.6
0.8
1
1.2

FIG. 6

(a)

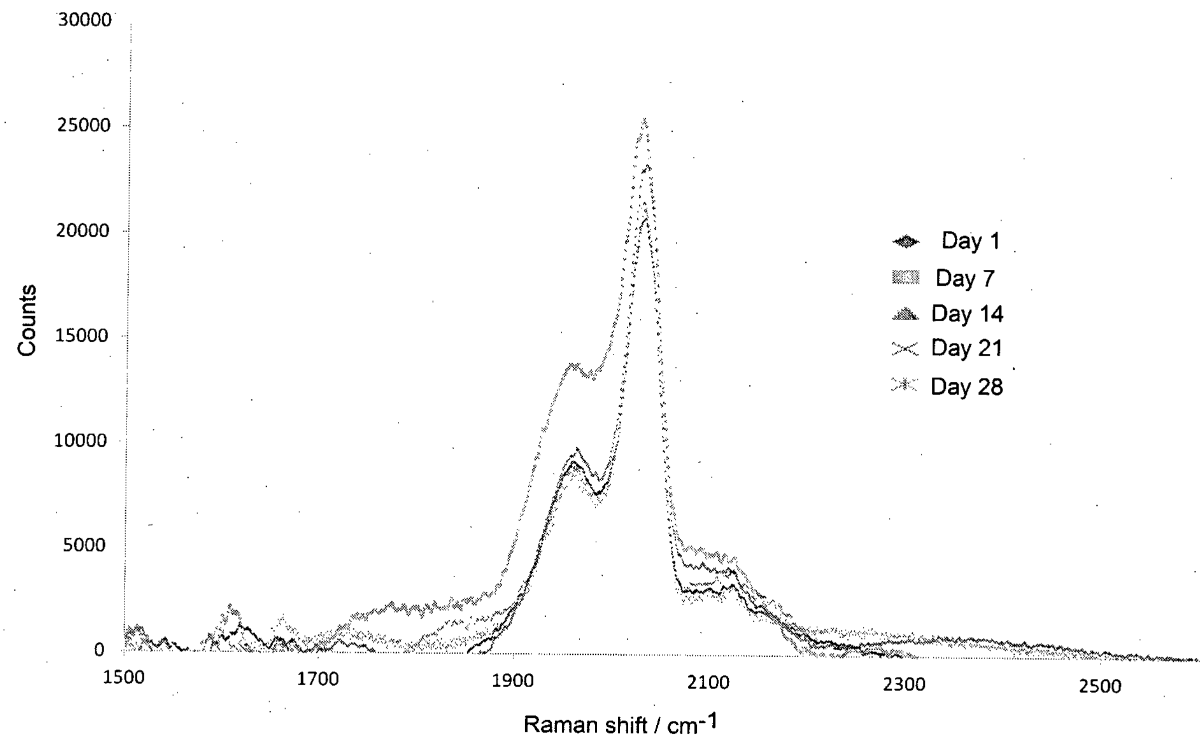
30000
25000
20000
15000
10000
5000
0
Counts
1500
1700
1900
2100
2300
2500
Raman shift / cm-1
Day 1
Day 7
Day 14
Day 21
Day 28

(b)

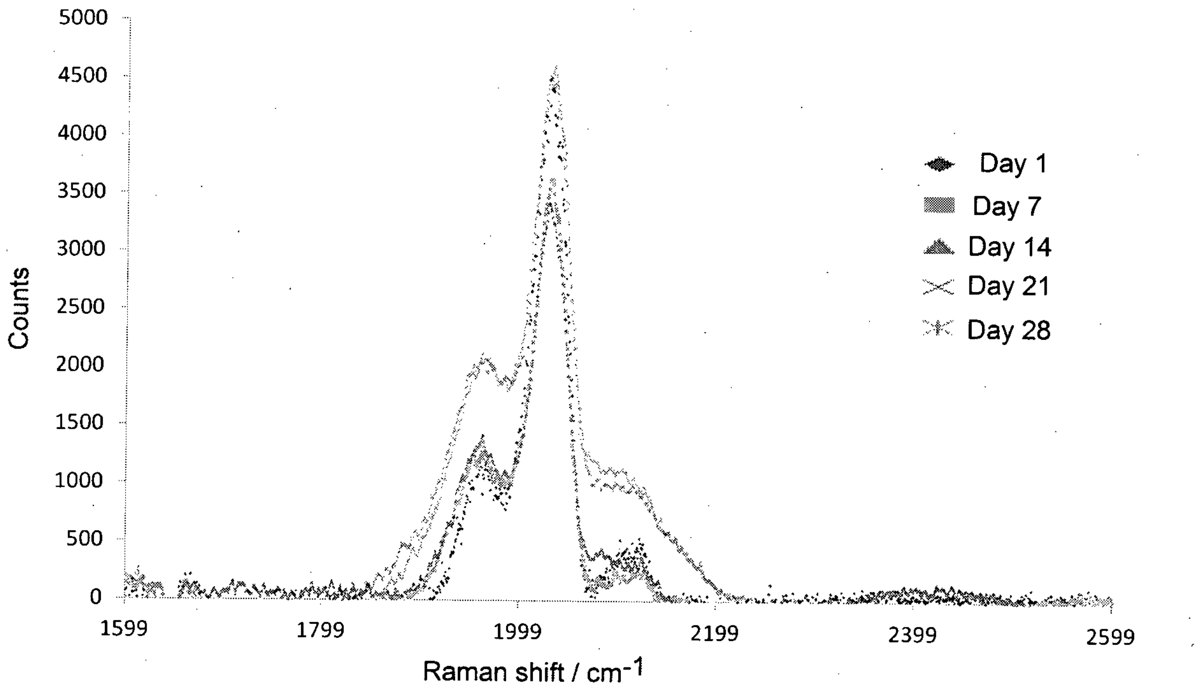
5000
4500
4000
3500
3000
2500
2000
1500
1000
500
0
Counts
1599
1799
1999
2199
2399
2599
Raman shift / cm-1
Day 1
Day 7
Day 14
Day 21
Day 28

FIG. 7

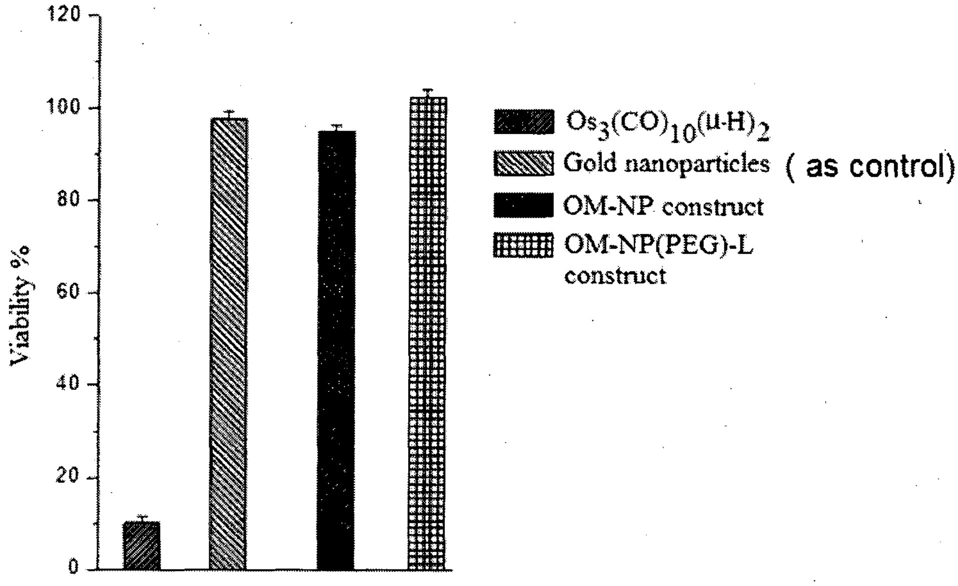
120
100
80
60
40
20
0
Viability %
$Os_3(CO)_{10}(\mu-H)_2$
Gold nanoparticles ( as control)
OM-NP construct
OM-NP(PEG)-L construct

FIG. 8

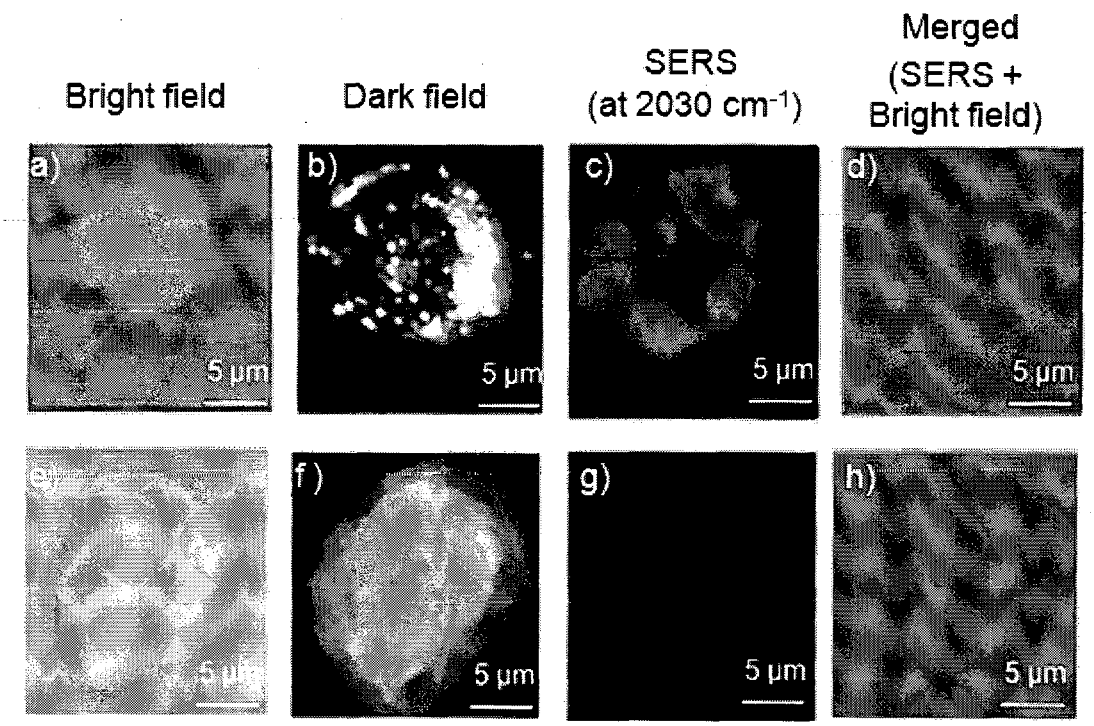
Bright field
Dark field
SERS (at 2030 $cm^{-1}$)
Merged (SERS + Bright field)
a)
b)
c)
d)
e)
f)
g)
h)
5 µm

FIG. 9

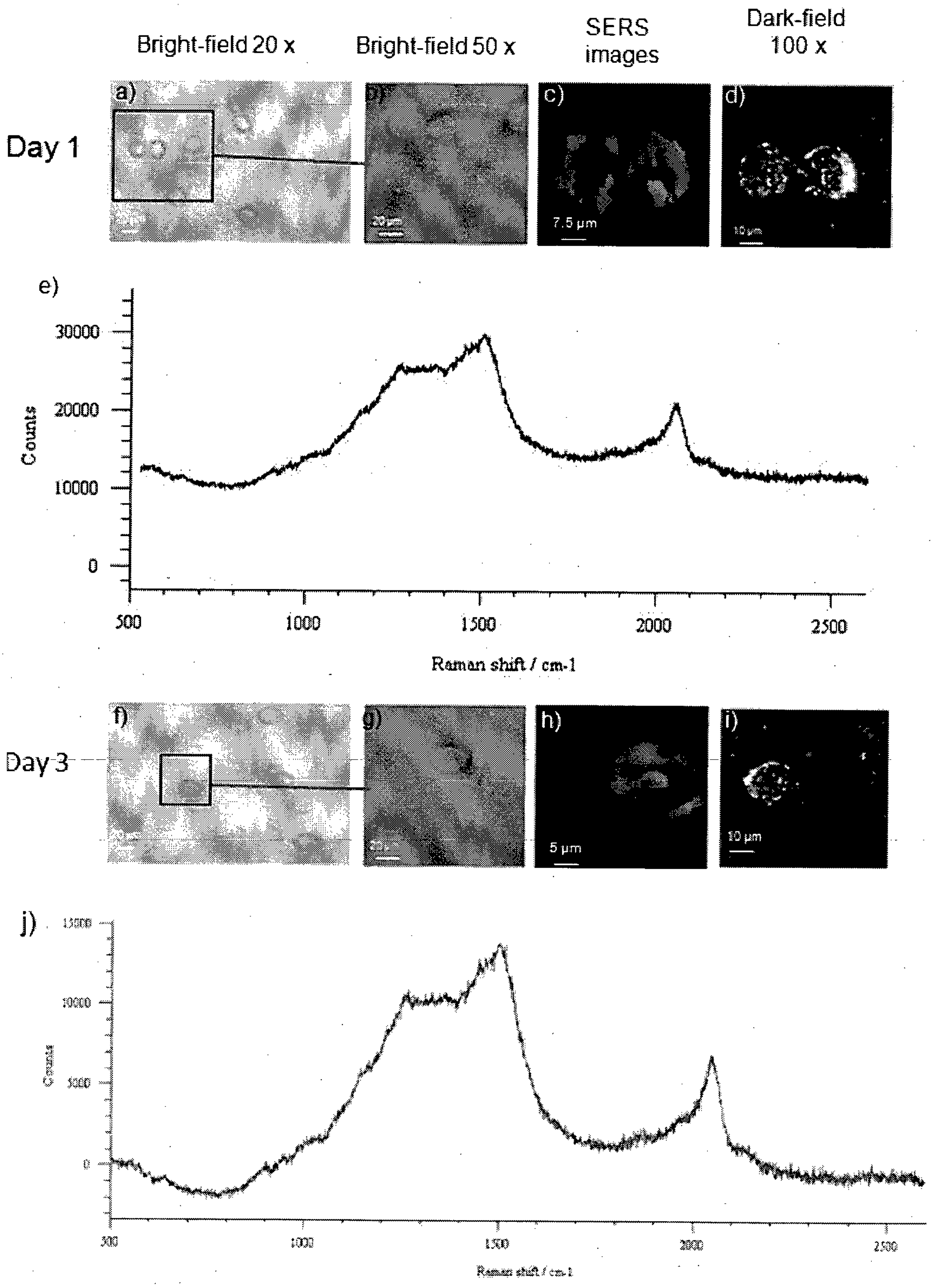

Bright-field 20 x
Bright-field 50 x
SERS images
Dark-field 100 x
Day 1
a)
b)
c)
d)
20 µm
7.5 µm
10 µm
e)
Counts
30000
20000
10000
0
500
1000
1500
2000
2500
Raman shift / cm-1
Day 3
f)
g)
h)
i)
5 µm
10 µm
j)
15000
10000
5000
0
Counts
500
1000
1500
2000
2500
Raman shift / cm-1

FIG. 10

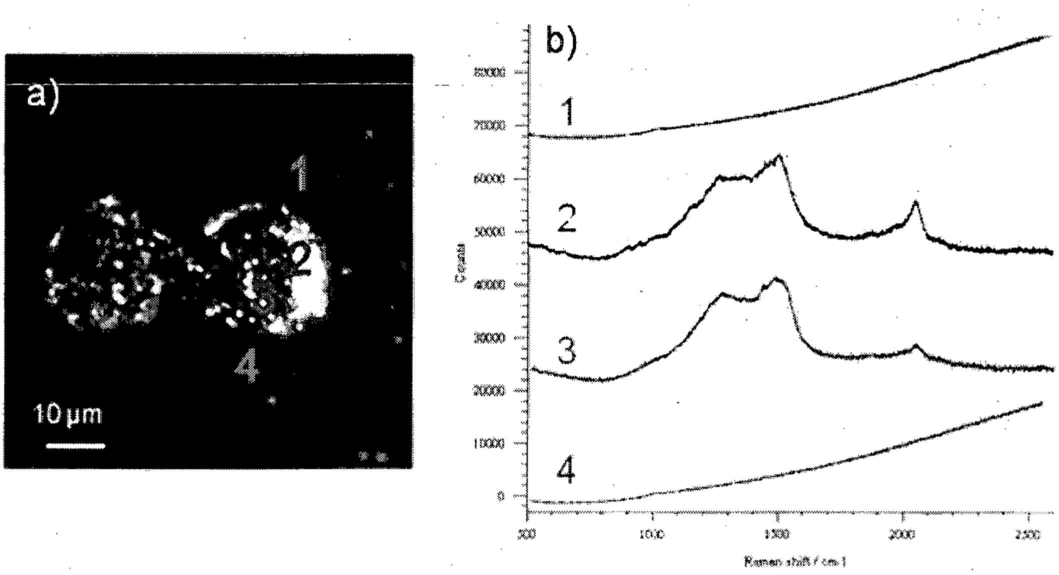
a)
1
2
4
10 µm
b)
1
2
3
4
Raman shift / cm-1

FIG. 11

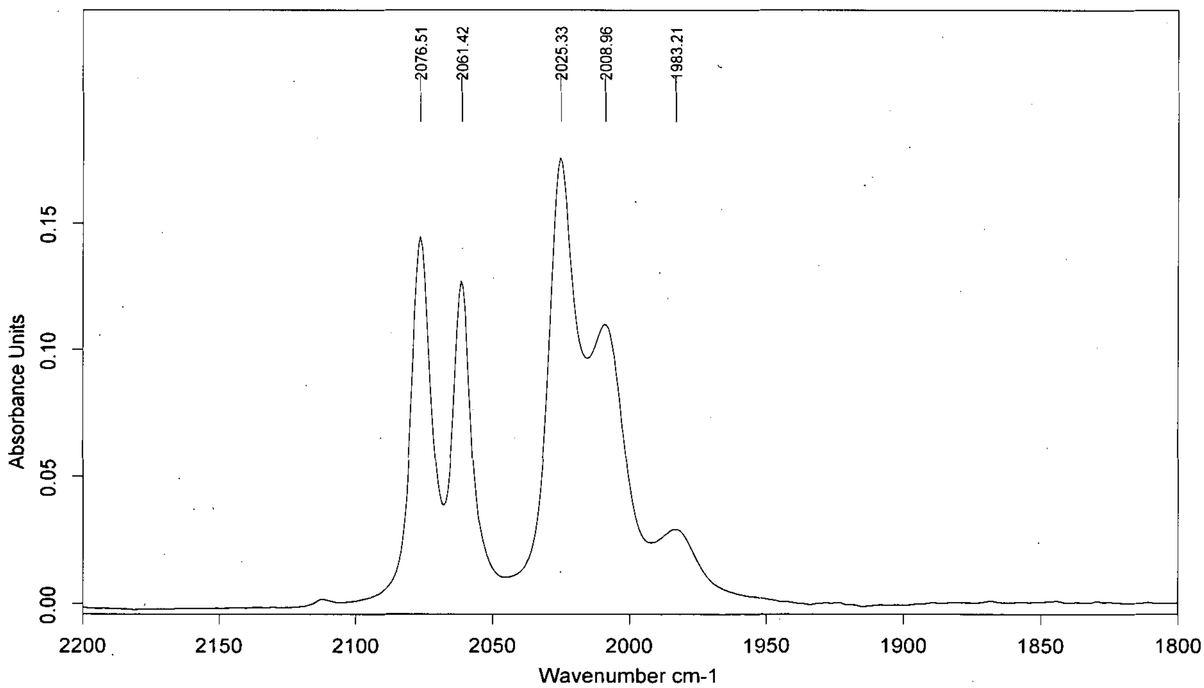
2076.51
2061.42
2025.33
2008.96
1983.21
Absorbance Units
0.00
0.05
0.10
0.15
2200
2150
2100
2050
2000
1950
1900
1850
1800
Wavenumber cm-1

FIG. 12

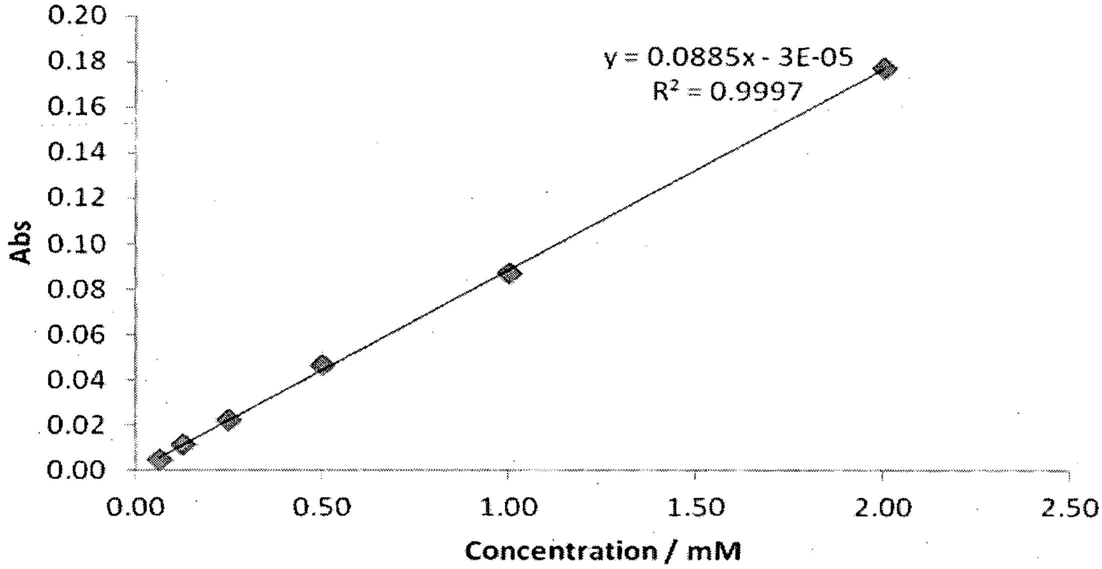


FIG. 13

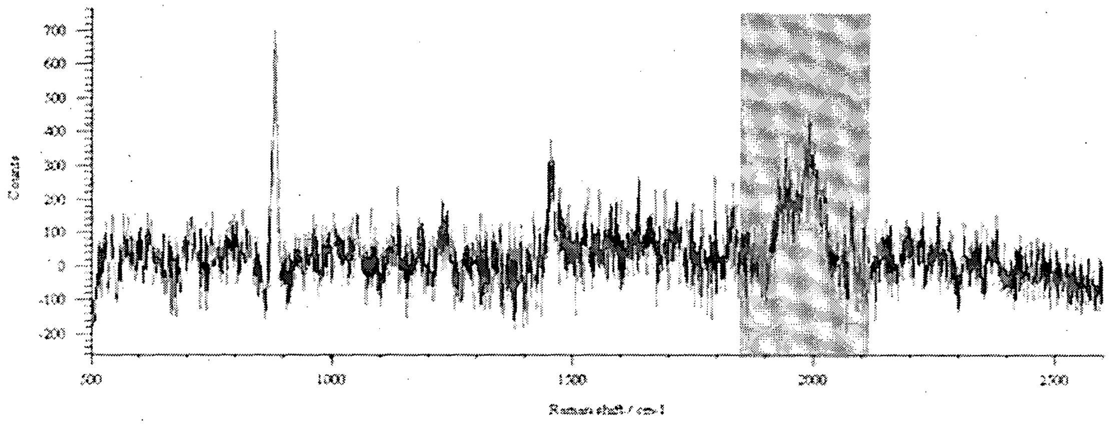


FIG. 14

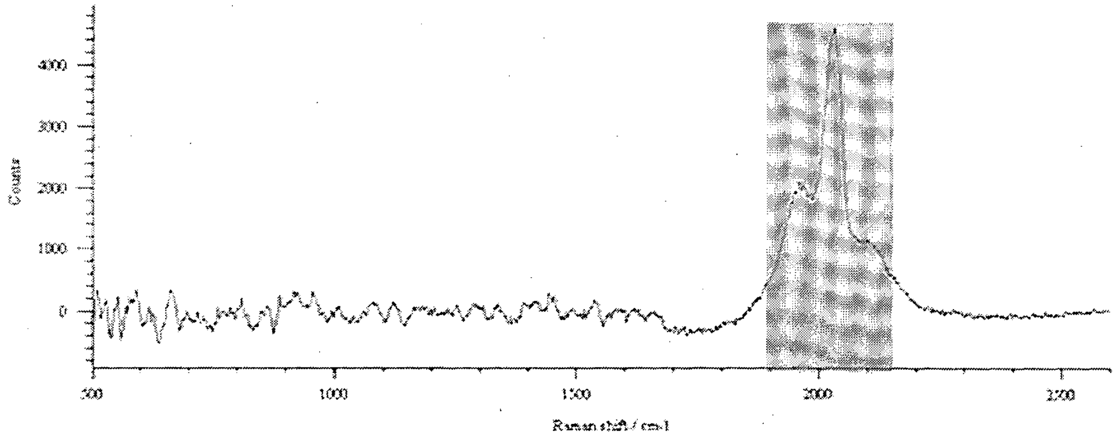

FIG. 15

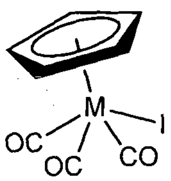

**1a:** M = Mo
**1b:** M = W

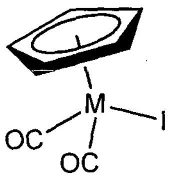

**2a:** M = Ru
**2b:** M = Os

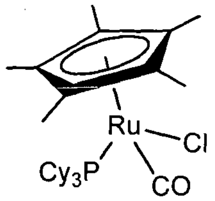

3

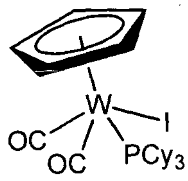

4

FIG. 16

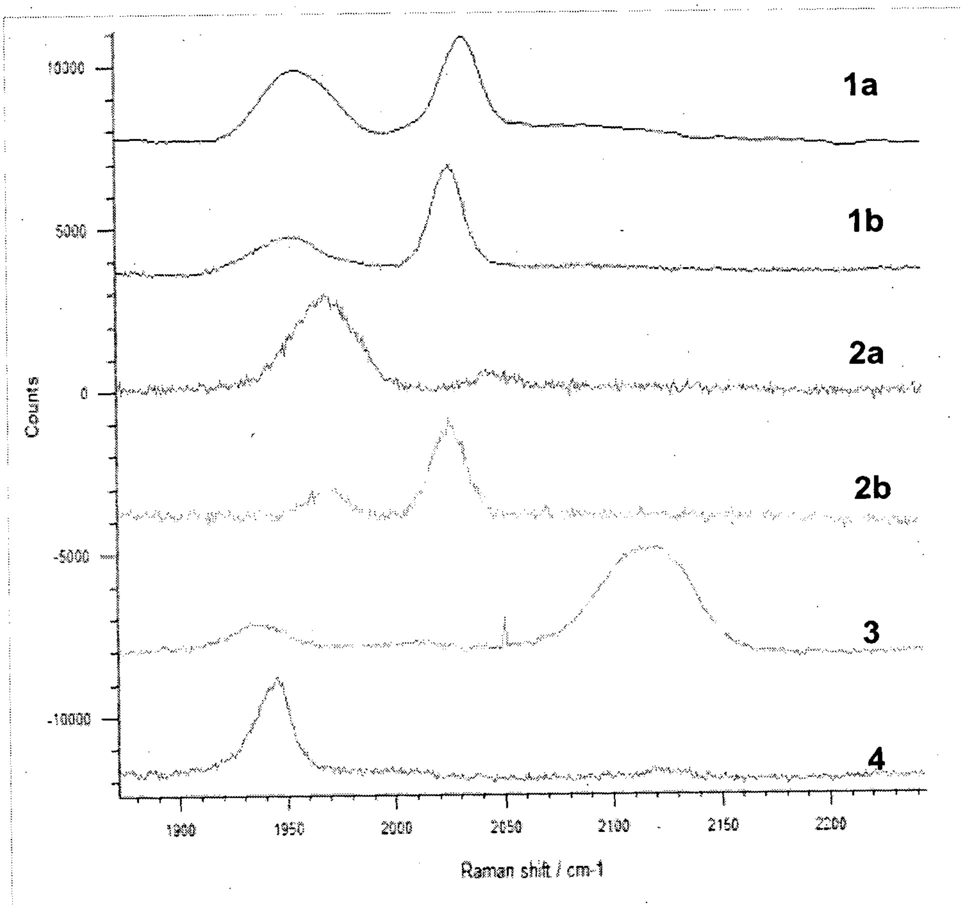

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120128592 A1
- US 4376110 A


### Non-patent literature cited in the description


- **LI, C. et al.** Osmium Carbonyl Clusters on Gold and Silver Nanoparticles as Models for Studying the Interaction with the Metallic Surface. *J. Phys. Chem. C,* 2009, vol. 113, 18562-18569
- **MONTGOMERY, H. J. et al.** *Examination of the Silver Colloid Binding Behavior of Disulfide-Tethered Bipyridine Ligands and Their fac-Tricarbonylrhenium(I) Complexes*
- **WANG, S. et al.** Au Nanoparticles Encapsulated in Ru Carbonyl Carboxylate Shells. *Langmuir,* 2006, vol. 22, 7861-7866
- **TAN, H. et al.** Preparation and Characterization of Cr(CO)4dpp (Chromium Tetracarbonyl 2,3-Bis(2'-pyridyl)pyrazine) Adsorbed on Silver Nanoparticles. *J. Phys. Chem. B,* 2005, vol. 109, 19657-19663
- **H. D. KAESZ.** *Inorg. Synth.,* 1990, vol. 28, 238-240